# EUROPEAN PATENT APPLICATION

(11) **EP 3 984 993 A1**
(43) Date of publication of application: **20.04.2022**
(21) Application number: 20821703.4
(22) Date of filing: 13.06.2020
(51) Int. Cl.: C07C 319/06, C07C 321/04, C07C 319/12, A61K 31/16, A61P 25/00, A61P 9/00

(54) **USE OF AMINOTHIOL COMPOUNDS AS CEREBRAL NERVE OR HEART PROTECTIVE AGENT**

(30) Priority: 13.06.2019 WO PCT/CN2019/091065
(71) Applicant: Shanghai Kechow Pharma, Inc., Shanghai 201203 (CN)
(72) Inventor: TIAN, Hongqi, Tianjin 300192 (CN); LIU, Yahong, Tianjin 300192 (CN)
(74) Representative: Viering, Jentschura & Partner mbB Patent- und Rechtsanwälte
(86) International application number: PCT/CN2020/095998
(87) International publication number: WO 2020/249120

(57) **Abstract**

Disclosed is the use of aminothiol compounds as a cerebral nerve or heart protective agent, in particular in the prevention and/or treatment of cardiovascular and cerebrovascular diseases, such as heart and cerebral ischemia/reperfusion injury and strokes.

## Description

### Field of the Invention

The present invention relates to the use of an aminothiol compound as a cerebral neuroprotective agent, especially the use in the prevention or treatment of cardio-cerebrovascular diseases, such as cardio-cerebrovascular ischemia reperfusion injury and stroke.

### Background of the Invention

Stroke is the leading cause of disability or death in humans worldwide. In some countries and cities, stroke has even surpassed cardiovascular disease as the first cause of death. According to the data from a sampling survey by China National Center for Biotechnology Development, it is showed that the annual incidence of cerebrovascular diseases in urban and rural areas of China is on average 200/100000, and the prevalence is 400-700/100000; in addition, there are 2.5 million new cases of cerebrovascular diseases nationwide each year, and the death rate is 130/100000, so cerebrovascular diseases are the second cause of death in the total population of China. Among stroke patients, 70-80% are ischemic stroke. For cerebral ischemic diseases, restoring the blood supply and oxygen supply in the brain tissue as soon as possible is one of the effective means to save their lives and reduce the disability rate; however, after the brain tissue restores blood oxygen supply, more serious damage appears in brain dysfunction, i.e., cerebral ischemia reperfusion injury (CIRI).

Cerebral ischemia reperfusion injury refers to the phenomenon that ischemic injury may be further aggravated when the blood supply in the brain tissue is restored after a period of time of ischemia. Cerebral ischemia reperfusion injury is clinically very common and difficult to avoid. After the blood stream in the brain tissue is restored, an ischemia reperfusion process necessarily occurs. Although restoring the blood circulation thereof is necessary to save the brain tissue, a series of complex reactions triggered by reperfusion may in turn aggravate the damage to the ischemic brain tissue. So far, there has been clinically no very effective method for preventing and treating cerebral ischemia reperfusion injury. Therefore, finding an effective measure to delay and alleviate cerebral ischemia reperfusion injury has become an important issue to be solved urgently.

At present, there have been a wide variety of drugs in a clinical application; however, the therapeutic effects thereof are not satisfactory. The treatment of stroke includes ultra-early thrombolysis and neuroprotective drugs. Early thrombolytic therapy is one of the most effective treatment methods for dredging blood vessels, reducing infarcts, and alleviating cerebral edema. However, thrombolytic therapy requires strict control of the treatment time window (within 6 hours of onset). Research data has shown that only 3-5% of acute ischemic stroke patients in the United States have received intravenous thrombolytic therapy; the National Survey of Stroke in China has shown that the intravenous rt-PA thrombolytic treatment rate is 1.6%. The reason for the low treatment rate is complex, and one of the important factors is the thrombolytic time window being beyond. Therefore, the application of thrombolysis is limited. Neuroprotection is another important means to treat ischemic stroke. After ischemia, the intracranial nerve cells will have a "cascade reaction" waterfall phenomenon, which provides a theoretical basis for the clinical application of neuroprotective agents. Since the 1950s, a large number of studies have been conducted at home and abroad on many aspects of the post-ischemia "cascade reaction" process, including calcium ion antagonists, anti-excitatory toxic amino acids, γ-aminobutyric acid receptor agonists, anti-intercellular adhesion molecule antibodies, neurotrophic drugs, etc. However, no positive research results from animal experiments have been confirmed in clinical trials.

Currently, drugs used for treating ischemic stroke, such as edaravone, were approved for marketing in Japan in 2001 and approved for marketing in China in 2005. However, since the compound did not achieve significant results in clinical trials in the United States and Europe, it was not approved by the drug registration authorities in the United States and Europe. In addition, edaravone has been disclosed to have serious side effects, such as acute renal function failure, abnormal hepatic function, jaundice, thrombocytopenia, and diffuse intravascular coagulation. At present, for the treatment of ischemic stroke, no effective neuroprotective agent has been approved for marketing by the drug registration departments of various countries.

CN 106432014A or WO 2018/041245 discloses aminothiol compounds, which are proven to be effective as drugs for protection and treatment against radiation damage; and compared with amifostine, these aminothiol compounds are considered to have longer half-lives and lower adverse reactions.

The inventors of the present invention have unexpectedly discovered that these aminothiol compounds can be used as cerebral neuroprotective agents for preventing or treating cardio-cerebrovascular diseases, such as stroke, especially ischemic stroke.

### Summary of the Invention

The inventors of the present invention have found that the aminothiol compound of the present invention has a significant remission rate in the cerebral infarction area compared with amifostine, which also has a protective effect on radiation damage, and therefore, the aminothiol compound of the present invention has a cerebral neuroprotective effect; in addition, compared with the positive drug edaravone, the aminothiol compound of the present invention also has a better remission rate in cerebral infarction areal and behavior.

In one aspect, the present invention provides the use of a compound of formula (I) or a compound selected from any one of the following Group (II) in the preparation of a medicament for use as a cerebral neuroprotective agent or cardioprotective agent, wherein the compound of formula (I) is 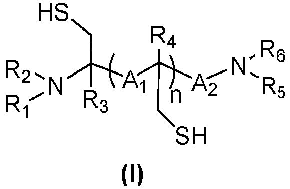 wherein
A₁ is selected from -C(O)NR⁸-, -S(O)₂-NR⁸-, -S(O)NR⁸-, and -R⁷-NR⁸-;
A₂ is selected from carbonyl, sulfonyl, sulfinyl, and substituted or unsubstituted C₁₋₆ alkyl;
R¹ and R⁵ may be the same or different and are selected from hydrogen, and substituted or unsubstituted C₁-C₅ alkyl or heteroalkyl;
R² and R⁶ may be the same or different and are selected from substituted or unsubstituted C₁-C₅ alkyl or heteroalkyl;
n is an integer of 0-20000;
R³ and R⁴ are independently selected from hydrogen, X, and substituted or unsubstituted C₁₋₆ alkyl;
X is selected from F, Cl, Br, and I;
R⁷ is selected from substituted or unsubstituted C₁-C₆ alkyl, and R⁸ is selected from hydrogen and substituted or unsubstituted C₁-C₆ alkyl;
or a stereoisomer thereof or a pharmaceutically acceptable salt, prodrug, and solvate thereof;
Compounds of Group (II): (R)-2-(((R)-3-mercapto-2-(methylamino)propyl)amino)-3-(me thylamino)propane-1-thiol or a hydrochloride thereof (compound 1), (R)-N-((R)-3-((2-meth oxyprop-2-yl)thio)-1-(methylamino)-1-oxopropane-2-yl)-2,2,3 -trimethylthiazolidine-4-carboxam ide (compound 2) and (R)-2,2,3-trimethyl-N-((6R, 9R)-3,3,12,12-tetramethyl-6-(methylcarba moyl)-8-oxo-2,13-dioxa-4,11-dithia-7-azatetradecan-9-yl)thiazolidine-4-carboxamide (compoun d 3), (R)-3-mercapto-N-((R)-3-mercapto-1-((2-(methylamino)ethyl)amino)-1-oxopropane-2-yl) -2-((2-(methylamino)ethyl)amino)propionamide, (R)-N-((7R,10R)-6,9-dioxo-13,13,13-triphenyl -7-(tritylthiomethyl)-12-thia-2,5,8-triazatridecan-10-yl)-3-mercapto-2-(2-(methylamino)ethylami no)propionamide, (R)-N-((R)-3,3-dimethyl-7-oxo-2-oxa-4-thia-8,11-diazadodecan-6-yl)-3-(2-m ethoxyprop-2-ylthio)-2-(2,2,3-trimethylimidazolidin-1-yl)propionamide and (R)-N-((R)-3,3-di methyl-7-oxo-2-oxa-4-thia-8,11-diazadodecan-6-yl)-3-(2-methoxyprop-2-ylthio)-2-((R)-3 -(2-me thoxyprop-2-ylthio)-2-(2,2,3-trimethylimidazolidin-1-yl)propionylamino)propionamide.

In one embodiment, the compound of formula (I) is (R)-3-mercapto-N-((R)-3-mercapto-1-(methylamino)-1-oxoprop-2-yl)-2-(methylamino)propionamide; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide.

In one embodiment, the compound of formula (I) is (R)-3-mercapto-N-((R)-3-mercapto-1-(methylamino)-1-oxoprop-2-yl)-2-(methylamino)propionamide or a pharmaceutically acceptable salt thereof; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide or a pharmaceutically acceptable salt thereof.

In one embodiment, the compound of formula (I) is (R)-3-mercapto-N-((R)-3-mercapto-1-(methylamino)-1-oxoprop-2-yl)-2-(methylamino)propionamide trifluoroacetate; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide trifluoroacetate.

In one embodiment, the compound of formula (I) is (R)-3-mercapto-N-((R)-3-mercapto-1-(methylamino)-1-oxoprop-2-yl)-2-(methylamino)propionamide hydrochloride; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide hydrochloride.

In one embodiment, the compound of formula (I) or the compound from Group (II) is used as a cerebral neuroprotective agent for the prevention or treatment of one or more of the following diseases or conditions: stroke, including ischemic and hemorrhagic stroke; secondary injury induced during the treatment of ischemic stroke, namely cerebral ischemia reperfusion injury; atherosclerosis, cerebral hemorrhage, hypertension, myocardial infarction, angina pectoris, coronary heart disease, myocardial fibrosis, heart disease, arrhythmia, dizziness, insomnia, etc.; varicose veins, edema and other diseases related to increased vascular permeability; senile diseases, such as Parkinson's disease, brain atrophy, and senile dementia; other brain nerve damage diseases, including but not limited to nerve injuries caused by brain trauma, sequelae of cerebrovascular sclerosis (cerebral hemorrhage, cerebral thrombosis), sequelae of encephalitis and meningitis, demyelinating diseases, ischemia, hypoxia, cerebrovascular accident, metabolic disorder, toxic effects, neurotoxic effects, surgery, iatrogenic effects, pressure, mass effect, hemorrhage, heat, chemical factors, radiation, vasospasm, neurodegenerative diseases, neurodegenerative lesions, infection, epilepsy, etc.; shock therapy, arterial bypass, thrombolytic therapy, percutaneous transluminal coronary angioplasty, cardiac surgery cardiopulmonary bypass, cardiopulmonary cerebral resuscitation, or limb replantation and organ transplantation; and systemic scleroderma, amyotrophic lateral sclerosis (ALS), etc.

In one embodiment, the compound of formula (I) or the compound from Group (II) is used as a cerebral neuroprotective agent for the prevention or treatment of stroke.

In one embodiment, the compound of formula (I) or the compound from Group (II) is used as a cerebral neuroprotective agent for the prevention or treatment of ischemic stroke.

In one embodiment, the compound of formula (I) or the compound from Group (II) is used as a cerebral neuroprotective agent for acute ischemic stroke.

In one embodiment, the compound of formula (I) or the compound from Group (II) is used as a therapeutic agent for the prevention or treatment of acute phase ischemic stroke and a post-ischemic-stroke cerebral neuroprotective agent.

In another embodiment, the compound of formula (I) or the compound from Group (II) is used as a cardioprotective agent for the prevention or treatment of myocardial ischemia, or myocardial infarction or cardio-cerebral ischemia reperfusion injury caused by myocardial ischemia.

In one embodiment, the dosage of the compound of formula (I) is about 9-16200 mg/kg/day.

In one embodiment, the present invention further relates to a novel aminothiol derivative selected from: (R)-2-(((R)-3-mercapto-2-(methylamino)propyl)amino)-3-(methylamino)propane-1-thiol or a hydrochloride thereof (compound 1), (R)-N-((R)-3-((2-methoxyprop-2-yl)thio)-1-(methylamino)-1-oxopropane-2-yl)-2,2,3-trimethylthiazolidine-4-carboxamide (compound 2) and (R)-2,2,3-trimethyl-N-((6R, 9R)-3,3,12,12-tetramethyl-6-(methylcarbamoyl)-8-oxo-2,13-dioxa-4,11-dithia-7-azatetradecan-9-yl)thiazolidine-4-carboxamide (compound 3), (R)-3-mercapto-N-((R)-3-mercapto-1-((2-(methylamino)ethyl)amino)-1-oxopropane-2-yl)-2-((2-(methylamino)ethyl)amino)propionamide, (R)-N-((7R,10R)-6,9-dioxo-13,13,13-triphenyl-7-(tritylthiomethyl)-12-thia-2,5,8-triazatridecan-10-yl)-3-mercapto-2-(2-(methylamino)ethylamino)propionamide, (R)-N-((R)-3,3-dimethyl-7-oxo-2-oxa-4-thia-8,11-diazadodecan-6-yl)-3-(2-methoxyprop-2-ylthio)-2-(2,2,3-trimethylimidazolidin-1-yl)propionamide and (R)-N-((R)-3,3-dimethyl-7-oxo-2-oxa-4-thia-8,11-diazadodecan-6-yl)-3-(2-methoxyprop-2-ylthio)-2-((R)-3-(2-methoxyprop-2-ylthio)-2-(2,2,3-trimethylimidazolidin-1-yl)propionylamino)propionamide.

In one embodiment, the aminothiol compound or aminothiol derivative of the present invention is used for the treatment and/or prevention of radiation damage, as a medicament for chemotherapeutic damage, and/or as a cosmetic. In one embodiment, the radiation includes ionizing radiation, non-ionizing radiation, or a combination of multiple types of radiation, wherein the ionizing radiation includes: α ray, β ray, γ ray, X-ray, and neutron radiation; and the radiation damage includes direct damage and indirect damage caused by radiation. Chemotherapeutic drugs refer to anti-tumor drugs that act on DNA, RNA, and tubulin.

In one embodiment, the aminothiol compound or aminothiol derivative of the present invention is used as an anti-tumor drug.

### Brief Description of the Drawings

Figure 1 shows the results of the neurobehavioral evaluation in rats after administration with compound A or compound B in an animal ischemic stroke model of Example 1.
Figure 2 shows the evaluation results of cerebral infarction area (TTC staining) in the rats after administration with compound A or compound B in the animal ischemic stroke model of Example 1.
Figure 3 shows the results of cerebral infarction area in the rats after administration with compound A or compound B in the animal ischemic stroke model of Example 1.
Figure 4 shows the results of the neurobehavioral evaluation in rats after administration with compound A, compound B, edaravone, or amifostine in an animal ischemic stroke model of Example 2.
Figure 5 shows the percent neurobehavioral improvement in rats after administration with compound A, compound B, edaravone, or amifostine in the animal ischemic stroke model of Example 2.
Figure 6 shows the results of cerebral infarction area in the rats after administration of compound A, compound B, edaravone, or amifostine in the animal ischemic stroke model of Example 2.
Figure 7 shows the percent improvement of cerebral infarction area in the rats after administration with compound A, compound B, edaravone, or amifostine in an animal ischemic stroke model of Example 2.
Figure 8 shows the evaluation results of cerebral infarction area (TTC staining) in the rats after administration with compound A, compound B, edaravone, or amifostine in an animal ischemic stroke model of Example 2.
Figure 9 shows the change in the body weight of cisplatin-induced SD rats by compound B/compound A in Example 3.
Figures 10A and 10B show the protective effect of compound B/A on cisplatin-induced nephrotoxicity in Example 3.
Figure 11 shows the protective effect of compound B on radiation intestinal injury in Example 4.
Figure 12 shows the protective effect of compound B on radiation lung injury.
Figure 13 shows the protective effect of compound B on radiation-induced hematopoietic system injury.
Figure 14 shows the survival rate of mice when compound 1 is administered.
Figure 15 shows the survival rate of mice when compound 2 is administered.
Figure 16 shows the survival rate of mice when compound 3 is administered.
Figure 17 shows the H-E staining of samples in skin lesion areas (×40).
Figure 18 shows the thickness of the dermis in the skin lesion area of the sample in each group.
Figure 19 shows the effects of compound A and B treatments on collagen in the skin lesion site.
Figure 20 shows the effects of compound A and B treatments on the H₂O₂ level in the skin lesion site.

### Detailed Description of Embodiments

In one aspect, the aminothiol compound involved in the present invention includes the compound of formula (I) disclosed in CN 106432014A or WO 2018/041245. In particular, the aminothiol compound involved in the present invention includes a compound of formula (I) 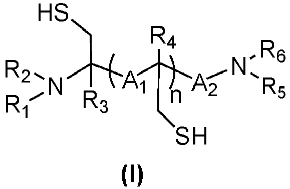 wherein
A₁ is selected from -C(O)NR⁸-, -S(O)₂-NR⁸-, -S(O)NR⁸-, and -R⁷-NR⁸-;
A₂ is selected from carbonyl, sulfonyl, sulfinyl, and substituted or unsubstituted C₁₋₆ alkyl;
R¹ and R⁵ may be the same or different and are selected from hydrogen, and substituted or unsubstituted C₁-C₅ alkyl or heteroalkyl;
R² and R⁶ may be the same or different and are selected from substituted or unsubstituted C₁-C₅ alkyl or heteroalkyl;
n is an integer of 0-20000;
R³ and R⁴ are independently selected from hydrogen, X, and substituted or unsubstituted C₁₋₆ alkyl;
X is selected from F, Cl, Br, and I;
R⁷ is selected from substituted or unsubstituted C₁-C₆ alkyl, and R⁸ is selected from hydrogen and substituted or unsubstituted C₁-C₆ alkyl;
or a stereoisomer thereof or a pharmaceutically acceptable salt, prodrug, and solvate thereof.

Preferably, R¹, R², R⁵, and R⁶ may be the same or different and are selected from hydrogen, C₁₋₃ alkyl, hydroxy or amino-substituted C₁-C₃ alkyl or heteroalkyl; more preferably, R¹, R², R⁵, and R⁶ may be the same or different and are selected from hydrogen, methyl, and ethyl; more preferably, one of R¹ and R² is hydrogen, the other is C₁-C₃ alkyl (for example, methyl, ethyl or propyl), and one of R⁵ and R⁶ is hydrogen, the other is C₁-C₃ alkyl (for example, methyl, ethyl or propyl); even more preferably, one of R¹ and R² is hydrogen, the other is methyl, and one of R⁵ and R⁶ is hydrogen, the other is methyl; alternatively, R¹ and R² are methyl or ethyl, and R⁵ and R⁶ are methyl or ethyl;
preferably, n is an integer of 0-2000; more preferably, n is an integer of 1-200; further preferably, n is an integer of 1-200; still further preferably, n is an integer of 1-50; more preferably, an integer of 1-10 (including 1, 2, 3, 4, 5, 6, 7, 8, 9, and 10);
preferably, R³ and R⁴are independently selected from hydrogen, X, and substituted or unsubstituted C₁₋₃ alkyl; more preferably, R³ and R⁴are independently selected from hydrogen, X, and methyl;
preferably, X is selected from F and Cl; more preferably, X is F;
preferably, R⁷ is selected from substituted or unsubstituted C₁-C₃ alkyl, and more preferably, R⁷ is methylene;
preferably, R⁸ is selected from hydrogen, and substituted or unsubstituted C₁-C₃ alkyl; more preferably, R⁸ is selected from hydrogen, methyl, and ethyl; further preferably, R⁸ is hydrogen; and
in the compound, the chiral carbons directly connected to R³ and R⁴ are in R configuration or S configuration. Preferably, the chiral carbons directly connected to R³ and R⁴ are both in R configuration.

More preferably, the chiral carbons directly connected to R³ and R⁴ are both in R configuration; and one of R¹ and R² is hydrogen, the other is methyl, and one of R⁵ and R⁶ is hydrogen, the other is methyl.

In one embodiment, the aminothiol compound of the present invention is (R)-3-mercapto-N-((R)-3-mercapto-1-(methylamino)-1-oxoprop-2-yl)-2-(methylamino)propionamide (abbreviated as compound A); and (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide (abbreviated as compound B).

In one embodiment, the aminothiol compound of the present invention includes the compound in a free base form or a pharmaceutically acceptable salt thereof. Examples include salts formed by the compound in the free base form with inorganic acids, such as hydrochloride, hydrobromide, nitrate, carbonate, bicarbonate, phosphate, monohydrogen phosphate, dihydrogen phosphate, sulfate, monohydrogen sulfate, hydriodate or phosphite; or salts formed with organic acids that are relatively free of toxic and side effects, such as acetate, trifluoroacetate, propionate, isobutyrate, oxalate, maleate, malonate, benzoate, succinate, suberate, fumarate, mandelate, phthalate, besylate, p-tosylate, citrate, tartrate, and mesylate.

In a preferred embodiment, the aminothiol compound of the present invention is trifluoroacetate of the above-mentioned compound A or compound B, or hydrochloride of compound A or compound B.

In one embodiment, the aminothiol compound of the present invention includes a prodrug form of the compound.

In one embodiment, the aminothiol compound of the present invention includes an unsolvated or solvated form of the compound, e.g., a hydrate form. Generally, the solvated form is equivalent to the unsolvated form, and both are included in the scope of the present invention. The compound of the present invention is present in a polymorphic or amorphous form. Generally, the various physical forms of the compound of the present invention are equivalent when used, and are all included within the scope of the present invention.

In another aspect, the aminothiol compound of the present invention includes the following novel aminothiol derivative, selected from (R)-2-(((R)-3-mercapto-2-(methylamino)propyl)amino)-3-(methylamino)propane-1-thiol or a hydrochloride thereof (compound 1), (R)-N-((R)-3-((2-methoxyprop-2-yl)thio)-1-(methylamino)-1-oxopropane-2-yl)-2,2,3-trimethylthiazolidine-4-carboxamide (compound 2) and (R)-2,2,3-trimethyl-N-((6R, 9R)-3,3,12,12-tetramethyl-6-(methylcarbamoyl)-8-oxo-2,13-dioxa-4,11-dithia-7-azatetradecan-9-yl)thiazolidine-4-carboxamide (compound 3), (R)-3-mercapto-N-((R)-3-mercapto-1-((2-(methylamino)ethyl)amino)-1-oxopropane-2-yl)-2-((2-(methylamino)ethyl)amino)propionamide, (R)-N-((7R,10R)-6,9-dioxo-13,13,13-triphenyl-7-(tritylthiomethyl)-12-thia-2,5,8-triazatridecan-10-yl)-3-mercapto-2-(2-(methylamino)ethylamino)propionamide, (R)-N-((R)-3,3-dimethyl-7-oxo-2-oxa-4-thia-8,11-diazadodecan-6-yl)-3-(2-methoxyprop-2-ylthio)-2-(2,2,3-trimethylimidazolidin-1-yl)propionamide and (R)-N-((R)-3,3-dimethyl-7-oxo-2-oxa-4-thia-8,11-diazadodecan-6-yl)-3-(2-methoxyprop-2-ylthio)-2-((R)-3-(2-methoxyprop-2-ylthio)-2-(2,2,3-trimethylimidazolidin-1-yl)propionylamino)propionamide.

The aminothiol compound involved in the present invention can be used as a cerebral neuroprotective agent. In the present application, the cerebral neuroprotective effect refers to reducing, stopping or eliminating nerve damage, and has protective, restorative and reactivating effects on nerve tissues suffering from nerve damage.

In particular, the aminothiol compound involved in the present invention can be used as a cerebral neuroprotective agent, and can be used for the prevention or treatment of one or more of the following diseases or conditions: stroke, including ischemic and hemorrhagic stroke; secondary injury induced during the treatment of ischemic stroke, namely cerebral ischemia reperfusion injury; atherosclerosis, cerebral hemorrhage, hypertension, myocardial infarction, angina pectoris, coronary heart disease, myocardial fibrosis, heart disease, arrhythmia, dizziness, insomnia, etc.; varicose veins, edema and other diseases related to increased vascular permeability; senile diseases, such as Parkinson's disease, brain atrophy, and senile dementia; other brain nerve damage diseases, including but not limited to nerve injuries caused by brain trauma, sequelae of cerebrovascular sclerosis (cerebral hemorrhage, cerebral thrombosis), sequelae of encephalitis and meningitis, demyelinating diseases, ischemia, hypoxia, cerebrovascular accident, metabolic disorder, toxic effects, neurotoxic effects, surgery, iatrogenic effects, pressure, mass effect, hemorrhage, heat, chemical factors, radiation, vasospasm, neurodegenerative diseases, neurodegenerative lesions, infection, epilepsy, etc.; shock therapy, arterial bypass, thrombolytic therapy, percutaneous transluminal coronary angioplasty, cardiac surgery cardiopulmonary bypass, cardiopulmonary cerebral resuscitation, or limb replantation and organ transplantation; and systemic scleroderma, amyotrophic lateral sclerosis (ALS), etc.

The aminothiol compound involved in the present invention, especially compound A or compound B, can be effective as a cerebral neuroprotective agent for the treatment or prevention of stroke, such as ischemic stroke or hemorrhagic stroke; and can be effective as a therapeutic agent for the prevention or treatment of acute phase ischemic stroke and as a post-ischemic-stroke cerebral neuroprotective agent. The treatment of the above-mentioned diseases is included in the scope of the present invention.

The aminothiol compound involved in the present invention, including the compound of formula (I) or the compound from Group (II), can be used as a cardioprotective agent for the prevention or treatment of myocardial ischemia or myocardial infarction caused by myocardial ischemia.

Myocardial ischemia is a metabolic disease that occurs when the coronary blood stream is insufficient to supply enough oxygen for consumption of carbon fuel and re-synthesis of ATP at normal rates. Ischemic myocardial ischemia may lead to an imbalance between the supply and demand of myocardial oxygen. Myocardial ischemia includes exertional and vasospastic myocardial dysfunctions. Exertional ischemia is generally due to the appearance of critical atherosclerotic stenosis, which includes reduced subendocardial flow caused by large coronary arteries. Vasospastic ischemia is related to spasm with lesion changes, and the onset thereof is not related to exertion and stress. Spasm is best defined as a sudden increase in vascular tension. The mechanism of vasospastic ischemia includes: (i) increased vascular tension at the stenosis caused by increased catecholamines; (ii) temporary intraluminal obstruction; and (iii) release of vasoactive substances formed by platelets at the endothelial injury. Coronary circulation is very unique because the circulation is distributed throughout organs that can generate full pressure for the entire circulation. Therefore, interferences that can change the condition of the peripheral circulation and the contraction force will have a huge effect on the coronary circulation. The regulating unit of the coronary vasculature is small coronary arteries whose inner diameters change greatly. The change in the inner diameter is due to the instinctive contraction (autoregulation) of vascular smooth muscle or the extravascular pressure caused by ventricular contraction. The pure therapeutic effect on ischemic diseases includes the combined effect of opposing factors that determine the supply and demand of oxygen.

The compound of formula (I) or the compound from Group (II) is used as a cardioprotective agent for improving ischemic injury or improving the extent of myocardial infarction attack or cardio-cerebral ischemia reperfusion injury.

"Improving ischemic injury" refers to preventing or reducing myocardial ischemic injury caused by myocardial ischemia. "Improving the extent of myocardial infarction attack" refers to reducing the extent of myocardial infarction attack caused by myocardial ischemia or preventing myocardial infarction caused by myocardial ischemia.

The aminothiol compound involved in the present invention may also be used for the prevention or treatment of the following diseases:
lung cancer, stomach cancer, breast cancer, rectal cancer, rhinitis cancer, etc.;
non-bacterial inflammation, such as femoral head necrosis, prostatitis, mastitis, etc.;
immune diseases, such as lupus erythematosus, psoriasis, scleroderma and other autoimmune diseases;
osteoarthropathy, such as arthritis, rheumatism, and rheumatoid;
cataracts, vitreous opacity, glaucoma, macular degeneration of the fundus, juvenile myopia, etc.;
diabetic nephropathy, diabetic eye disease, etc.; and
avian influenza infection, sepsis, femoral head necrosis, prostatitis, mastitis, lupus erythematosus, psoriasis, scleroderma, arthritis, rheumatism, rheumatoid, cataract, vitreous opacity, glaucoma, macular degeneration of the fundus, juvenile myopia, dry eye syndrome, diabetes and complications thereof (diabetic nephropathy, and diabetic eye disease), interstitial lung disease, idiopathic pulmonary fibrosis, sarcoidosis, chronic obstructive pulmonary disease (COPD), emphysema, amyotrophic lateral sclerosis (ALS), senile plaques, wrinkles, renal ischemia reperfusion injury in kidney transplantation, periodontitis, bronchial asthma, allergic asthma, obstructive sleep apnea, pain, postoperative myalgia, obesity, diaphragm dysfunction mechanical ventilation, cytomegalovirus infection, hyperlipidemia, acute pancreatitis, pancreatic fibrosis, hepatitis, cirrhosis, liver fibrosis, osteoporosis, Fanconi's anemia, peripheral artery occlusive disease, endometriosis, polycystic ovarian syndrome, asthenozoospermia, acute mountain sickness, etc.

The aminothiol compound involved in the present invention or a pharmaceutically acceptable salt or hydrate thereof can be administered through various routes of administration. Suitable routes of administration include, but are not limited to, inhalation administration, or parenteral routes, including intravenous administration (infusion or bolus administration), intramuscular administration, intraperitoneal administration, intrathecal administration, subcutaneous administration, intranasal administration, transmucosal administration, sublingual administration, enteral administration, and topical administration. Preferably, the aminothiol compound involved in the present invention is administered through oral route or intravenously.

The aminothiol compound involved in the present invention or a pharmaceutically acceptable salt and/or hydrate thereof can be administered to a subject alone, or the aminothiol compound involved in the present invention or a pharmaceutically acceptable salt and/or hydrate thereof is administered to the subject in combination with one or more other agents (including but not limited to other cerebral neuroprotective agents, anti-tumor agents, chemotherapeutics, etc.).

When administering the aminothiol compound of the present invention, the range of the dosage of administration is usually about 9-16200 mg/kg/day, generally about 9-8100 mg/kg/day, or about 9-4000 mg/kg/day, or about 9-2000 mg/kg/day, or about 9-1000 mg/kg/day, or about 9-500 mg/kg/day, or about 9-250 mg/kg/day, or about 9-120 mg/kg/day, or about 9-50 mg/kg/day, or about 9-40 mg/kg/day, or about 9-20 mg/kg/day, based on the weight of the patient.

The specific mode of administration and dosage and interval of administration will depend on the body weight of the subject, the severity of the disease, the mode of administration, and the judgment of the prescribing physician.

### Example

The following examples are used to illustrate the present invention, but the present invention is not limited thereto.

### Example 1: Evaluation of the effect of compound A or compound B in an ischemic stroke model

### 1. Experimental animal

Healthy male SPF grade SD rats (body weight 250-300 g) were selected.

### 2. Experimental grouping (5 groups * 6 rats)

The selected rats were divided into the following groups (five groups: groups A, B, C, D, and E, with 6 rats in each group):

| | |
|---|---|
| Group A | Blank control group |
| Group B | Ischemia-reperfusion modeling group |
| Group C | Administered with compound B (200 mg/kg i.p.) 5 min before reperfusion |
| Group D | Administered with compound B (200 mg/kg i.p.) during ischemia and with compound B (200 mg/kg i.p.) 5 min before reperfusion |
| Group E | Administered with compound A (1600 mg/kg p.o.) intragastrically 2.5 h before reperfusion |

| | |
|---|---|
| Note: Compound A and compound B used in the examples were both in the form of trifluoroacetate thereof | |

Compound B was dissolved in phosphate buffered saline (PBS) and then stored under refrigeration. It was prepared on the spot when needed to avoid exposure to air and light. Compound A was dissolved in a solvent of 15% Solutol HS 15 (obtained from Shenzhen Haobo Shiji Shengwu Youxian Gongsi)/85% HP-B-CD (obtained from Solarbio) (20%, w/v), and the storage method was the same as that for compound B.

### 3. Modeling method

(1) The rats were anesthetized, the skin on the neck was prepared, and the body temperature was maintained at 37±0.5°C.
(2) A midline neck incision was made to expose the right common carotid artery, internal carotid artery and external carotid artery. A 6-0 silk thread was used to ligate the distal end of the external carotid artery at 4 mm from the bifurcation of the common carotid artery, another 6-0 silk thread was inserted at the external carotid artery, and a slipknot was tied near the bifurcation of the common carotid artery.
(3) An arterial clamp was used to clamp the common carotid artery. A small opening was cut on the external carotid artery at 3 mm from the bifurcation of the common carotid artery, and a 0.33 mm diameter nylon thread with a treated head end was inserted through the small opening, entered the internal carotid artery, and was inserted inward to the middle cerebral artery, wherein the insertion depth of the nylon thread was about 16±1 mm from the bifurcation of the common carotid artery.
(4) The thread embolism was removed 90 min after ischemia, the proximal end of the external artery was ligated with a 6-0 silk thread, the neck wound was sutured with a 3-0 silk thread, the wound was disinfected with povidone iodine, the rats were placed on a heating pad, and after being awake, they were placed in a constant temperature rearing box for rearing.
(5) 24 h after operation, the rats were scored for neurological function, the rats were then anesthetized, and the brains were taken and stained with TTC (2,3,5-triphenyltetrazolium chloride, Sigma) as described below.

### 4. Neurobehavioral evaluation of the model

The experimental animals were scored for signs of neurological function deficit before and after modeling. The scoring standard was given by reference to the 5-point system of Longa and Bederson. The scoring was performed 24 h after the animals were awake from anesthesia. The higher the score, the more severe the behavioral disorder of the animals.
0 point: No nerve damage symptoms
1 point: Inability to fully extend the contralateral front paw
2 points: Turning in a circle to the opposite side
3 points: Falling over to the opposite side
4 points: Inability to walk spontaneously, and loss of consciousness

### 5. TTC staining and infarction area statistics (5 groups * 6 rats)

After the rats were anesthetized, the brain tissues were taken from the rats, placed in a refrigerator and frozen at -20°C for 30 min. 1% TTC (W/V) was prepared with PBS and placed in a water bath at 37°C until the TTC was dissolved, and the frozen brain tissue was sliced, placed in 10 ml of the TTC solution and incubated at 37°C for 10 min. The brain slices were turned from time to time to make the tissue evenly stained. Normal brain tissue was bright red after staining, whereas the infarction region was pale white in color or light white in a black and white image. In addition, a software was used to measure and statistically analyze the infarction area.

### 6. Experimental results

The following table summarized the behavioral score and cerebral infarction area data of each animal

| No. | Neurological function score | | | Cerebral infarction area (mm³) | % Improvement of cerebral infarction area |
|---|---|---|---|---|---|
| | Before modeling | After modeling | % Behavioral improvement | | |
| A1 | 0 | 0 | ------ | 0 | ------ |
| A2 | 0 | 0 | | 0 | |
| A3 | 0 | 0 | | 0 | |
| A4 | 0 | 0 | | 0 | |
| A5 | 0 | 0 | | 0 | |
| A6 | 0 | 0 | | 0 | |
| B1 | 0 | 2 | | 159.21484 | |
| B2 | 0 | 3 | | 108.1214 | |
| B3 | 0 | 4 | | 199.97271 | |
| B4 | 0 | 3 | | 143.716952 | |
| B5 | 0 | 3 | | 159.42731 | |
| B6 | 0 | 3 | | 169.43521 | |
| C1 | 0 | 0 | 66.7% | 67.443139 | 62.5% |
| C2 | 0 | 1 | | 67.69023 | |
| C3 | 0 | 1 | | 32.83715 | |
| C4 | 0 | 1 | | 23.399732 | |
| C5 | 0 | 2 | | 105.904392 | |
| D1 | 0 | 1 | 66.7% | 67.530303 | 44.4% |
| D2 | 0 | 1 | | 105.501335 | |
| E1 | 0 | 1 | 77.8% | 40.191728 | 83.9% |
| E2 | 0 | 1 | | 26.33 | |
| E3 | 0 | 0 | | 9.18 | |

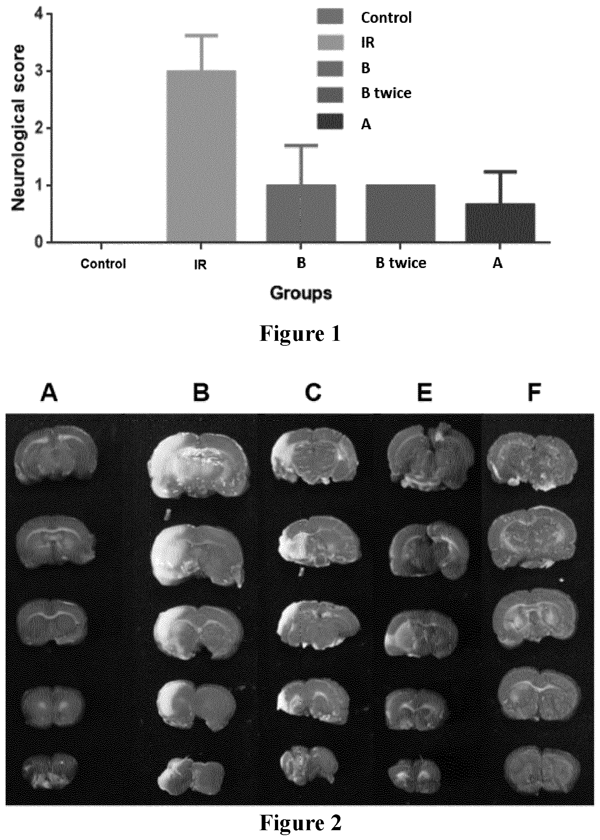

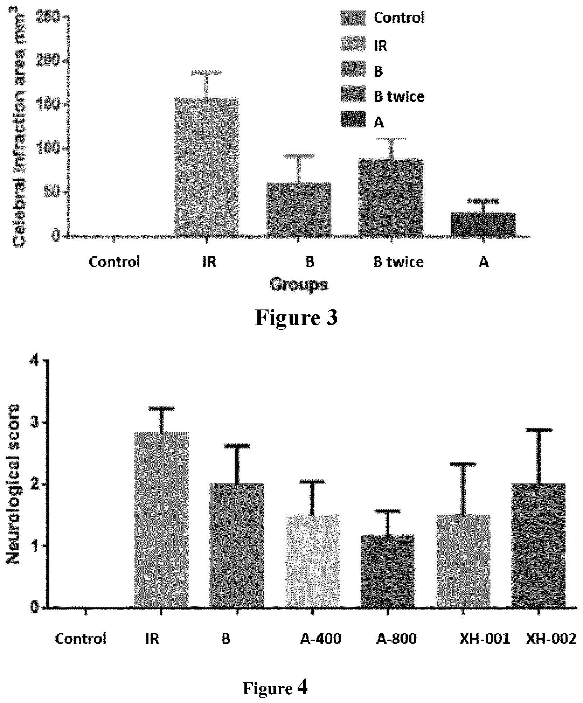

The above results indicated that compound A or compound B had an excellent improvement effect on the animal ischemic stroke model (MCAO), and both significantly improved the ischemia reperfusion injury in the model animal in terms of either the cerebral infarction area evaluation or the behavioral score. For example, the percent neurological improvements in group C, group D, and group E were respectively 66.7%, 66.7%, and 77.8% (see Figure 1); and the percent improvements of cerebral infarction area were respectively 62.5%, 44.4% and 83.9% (see Figures 2 and 3).

### Example 2: Evaluation of the effects of compound B, compound A, amifostine, and edaravone on SD rat cerebral ischemia reperfusion injury model

In a manner same as in Example 1, experimental animals were selected, except that the experiment was divided into seven groups (i.e., groups A, B, C, D, E, F, and G, among which groups C, D, and E were given different dosages of administration from Example 1, group F was given edaravone, and group G was given amifostine). Then, modeling was performed in the same manner as in Example 1, neurobehavioral evaluation was performed on the 7 groups of experimental models in the same manner, and TTC staining and infarction area statistics were performed on the 7 groups of experiments in the same manner.

The experiment groups in Example 2 were as follows:

| | |
|---|---|
| Group A | Blank control group |
| Group B | Ischemia-reperfusion modeling group |
| Group C | Administered with compound B (100mg/kg i.p.) 5 min before reperfusion |
| Group D | Administered with compound A (400 mg/kg p.o.) 2.5 h before reperfusion |
| Group E | Administered with compound A (800 mg/kg p.o.) 2.5 h before reperfusion |
| Group F | Administered with edaravone (XH-001) (3 mg/kg, i.v.) before reperfusion |
| Group G | Administered with amifostine (XH-002) (100 mg/kg, i.p.) 5 min before reperfusion |

| | |
|---|---|
| Note: Compound A and compound B used in the examples were both in the form of trifluoroacetate thereof | |

Compound B was dissolved in phosphate-buffered saline (PBS) and then stored under refrigeration. It was prepared on the spot when needed to avoid exposure to air and light. Compound A was dissolved in a solvent of 15% Solutol HS 15/85% HP-B-CD (20%, w/v), and the storage method was the same as that for compound B. Edaravone was dissolved in IN NaOH and titrated to 7.4 with IN HCl; and amifostine was dissolved in PBS.

The following table summarized the behavioral score and cerebral infarction area data of each animal

| **No.** | **Neurological function score** | | | **Cerebral infarction area (mm³)** | **% Improvement of cerebral infarction area** |
|---|---|---|---|---|---|
| | **Before modeling** | **After modeling** | **% Behavioral improvement** | | |
| **A1** | **0** | **0** | **-------** | **0** | **-------** |
| **A2** | **0** | **0** | | **0** | |
| **A3** | **0** | **0** | | **0** | |
| **A4** | **0** | **0** | | **0** | |
| **A5** | **0** | **0** | | **0** | |
| **A6** | **0** | **0** | | **0** | |
| **B1** | **0** | **3** | **-------** | **128.25** | **-------** |
| **B2** | **0** | **3** | | **156.71** | |
| **B3** | **0** | **3** | | **106.28** | |
| **B4** | **0** | **3** | | **168.497** | |
| **B5** | **0** | **2** | | **20.707** | |
| **B6** | **0** | **3** | | **134.271** | |
| **C1** | **0** | **1** | **29.32%** | **0** | **59.5%** |
| **C2** | **0** | **2** | | **32.568** | |
| **C3** | **0** | **2** | | **45.956** | |
| **C4** | **0** | **2** | | **21.529** | |

| | | | | | |
|---|---|---|---|---|---|
| **C5** | **0** | **3** | | **104.008** | |
| **C6** | **0** | **2** | | **85.208** | |
| **D1** | **0** | **2** | **46.99%** | **25.16** | **72.97%** |
| **D2** | **0** | **1** | | **11.23** | |
| **D3** | **0** | **1** | | **20.59** | |
| **D4** | **0** | **1** | | **15.48** | |
| **D5** | **0** | **2** | | **38.04** | |
| **D6** | **0** | **2** | | **82.75** | |
| **E1** | **0** | **1** | **59.01%** | **14.23** | **87.32%** |
| **E2** | **0** | **1** | | **0** | |
| **E3** | **0** | **1** | | **16.93** | |
| **E4** | **0** | **1** | | **12.31** | |
| **E5** | **0** | **2** | | **47.18** | |
| **E6** | **0** | **1** | | **0** | |
| **F1** | **0** | **1** | **46.99%** | **0** | **58.95%** |
| **F2** | **0** | **1** | | **10.31** | |
| **F3** | **0** | **2** | | **45.35** | |
| **F4** | **0** | **3** | | **220.66** | |
| **F5** | **0** | **1** | | **17.11** | |
| **F6** | **0** | **1** | | **0** | |
| **G1** | **0** | **2** | **29.32%** | **58.88** | **23.24%** |
| **G2** | **0** | **2** | | **20.71** | |
| **G3** | **0** | **1** | | **0** | |
| **G4** | **0** | **3** | | **244.96** | |
| **G5** | **0** | **3** | | **204.11** | |
| **G6** | **0** | **1** | | **20.31** | |

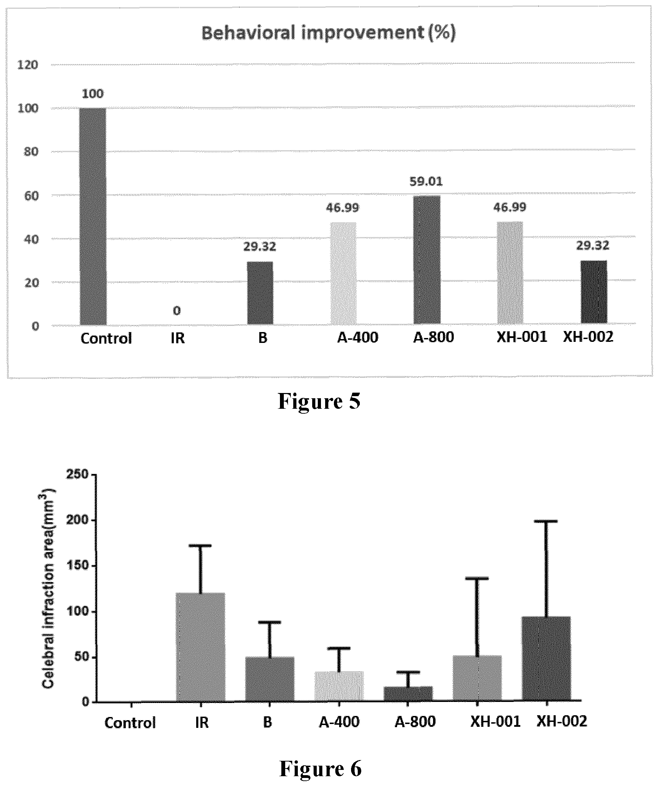

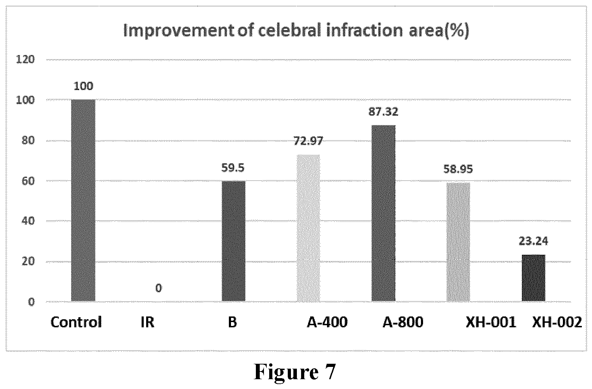

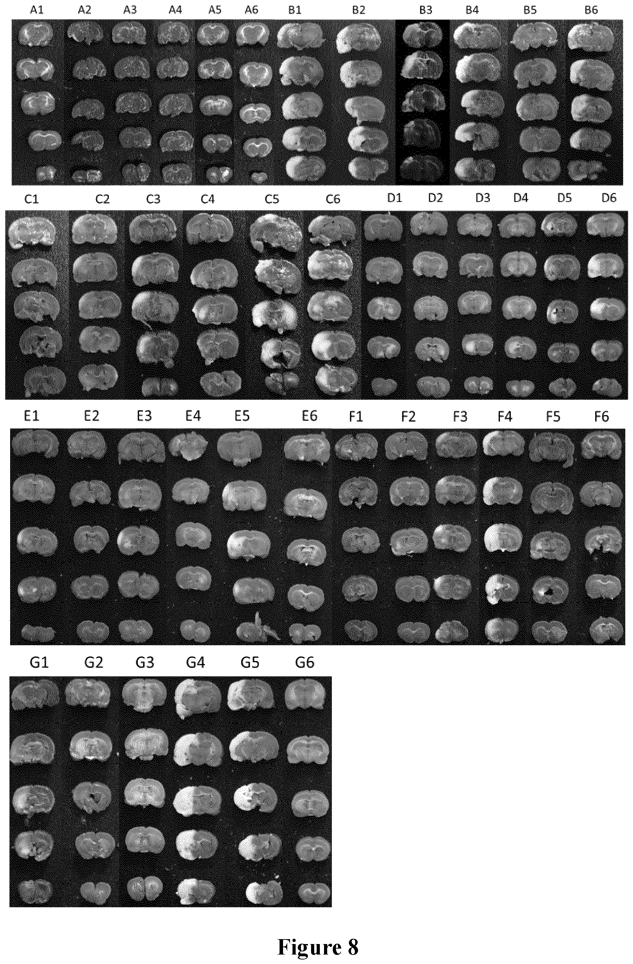

The above results indicated that the percent neurological improvements in groups C, D, E, F, and G were respectively 29.32%, 46.99%, 59.01%, 46.99%, and 29.32%, (see Figures 4 and 5); and the percent improvements of cerebral infarction area were respectively 59.5%, 72.97%, 87.32%, 58.85%, and 23.24% (see Figures 6, 7 and 8). Furthermore, when the dosage of compound B was reduced to 100 mg/kg, there remained a good protective effect in the rat MCAO model. In addition, after the dosage of compound A was reduced to 800 and 400 mg/kg, the symptoms and cerebral infarction area of the MCAO rats were significantly alleviated in a dose-dependent manner. Compared with the positive drug XH-001 (edaravone), compound A had a better effect, with a cerebral infarction area remission rate of up to 87.32%, whereas amifostine had the worst effect, with a cerebral infarction area remission rate of only 23.24%.

### Example 3: Protective effect of compound B/compound A on cisplatin-induced nephrotoxicity in SD rats

### 1. Experimental animal

Healthy male SPF grade SD rats (body weight 250-300 g) were selected.

### 2. Experimental protocol

Control group: PBS
Cisplatin: 5 mg/kg i.p.
Cisplatin + compound B group: 5 mg/kg i.p. + compound B at 200 mg/kg i.p., with compound B being administered 30 min prior to cisplatin administration
Cisplatin + amifostine group: 5 mg/kg i.p. + AMI at 200 mg/kg i.p., with AMI being administered 30 min prior to cisplatin administration
Cisplatin + compound A group: 5 mg/kg i.p. + compound A at 800 mg/kg p.o., with compound A being administered 30 min prior to cisplatin administration

### 3. Experimental steps

(1) The SD rats used in the experiment were weighed, labeled, and evenly divided into 5 groups, with 3 SD rats in each group.
(2) The compound B, compound A, and AMI groups were respectively administered at the planned concentrations. After half an hour, the cisplatin group, compound B group, compound A group, and amifostine group were respectively given cisplatin at 5 mg/kg, and the control group was given PBS.
(3) The rats were weighed and recorded daily, and on day 5, blood was taken from the intraocular canthus of the SD rats and placed in an anticoagulant tube.
(4) 100 µL of whole blood was taken in each tube, placed in a Smart reagent tray, and put into a fully automatic biochemical analyzer (SMT-100V) for detecting the levels of blood creatinine and urea nitrogen in the whole blood.

### 4. Experimental results

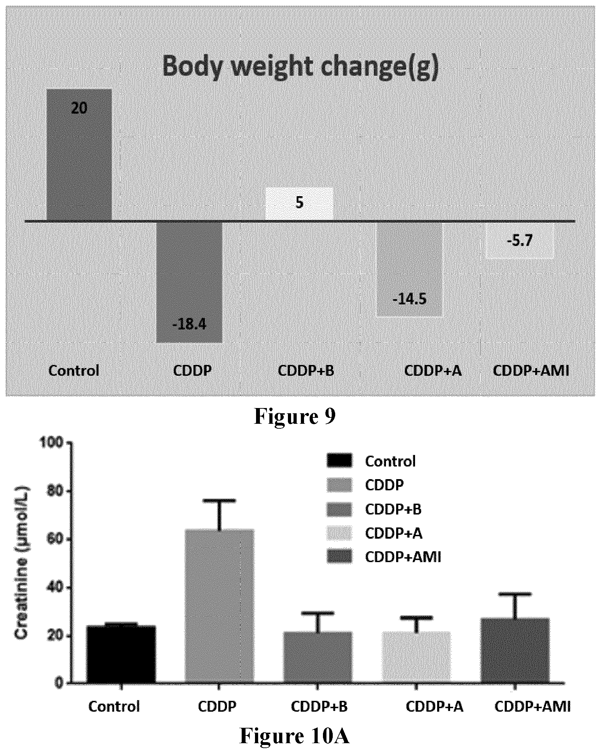

The changes in the body weight of the SD rats were shown in Figure 9. When the SD rats were given cisplatin alone, the body weight dropped significantly, and when compound B was administered, the body weight increased rather than decrease; and compared with the cisplatin group, although the body weights of the rats in the compound A group and the amifostine group decreased, the degree of decrease was significantly better than that of the cisplatin group, indicating that compound B and compound A had certain preventive effects on cisplatin-induced weight loss in rats.

### (2) Changes in blood creatinine and urea nitrogen levels

From the table and drawings, we could see that cisplatin could significantly increase the levels of creatinine and urea nitrogen in the blood of the rats, while compound A, compound B and amifostine could all significantly reduce the increased blood creatinine and urea nitrogen levels induced by cisplatin, and the activities of compound B and compound A were even slightly stronger than that of amifostine.

| Group | Creatinine (µmol/L) | Urea nitrogen (mmol/L) |
|---|---|---|
| Control group | 25 | 4.74 |
| | 23.5 | 5.11 |
| | 22.2 | 4.61 |
| 5 mpk CDDP | 58.6 | 18.70 |
| | 54.1 | 7.81 |
| | 78 | 12.9 |
| 200 mpk compound B +5 mpk CDDP | 30.4 | 6.77 |
| | 18.1 | 7.28 |
| | 14.7 | 5.71 |
| 800 mpk compound A +5 mpk CDDP | 28.4 | 7.68 |
| | 18.1 | 7.16 |
| | 17.1 | 10.21 |
| 200 mpk amifostine +5 mpk CDDP | 19.8 | 5.51 |
| | 38.9 | 5.34 |
| | 21.9 | 5.49 |

| | | |
|---|---|---|
| Note: Compound A and compound B used in the examples were both in the form of trifluoroacetates thereof | | |

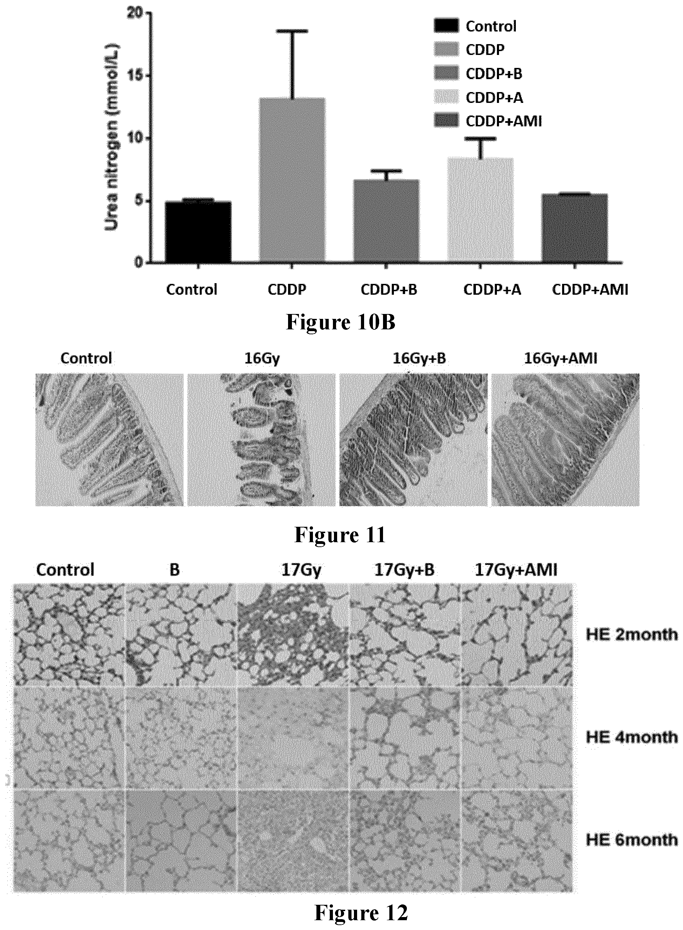

As shown in the above table and Figures 10A and 10B, compound B (TFA)/compound A (TFA) had good protective effects on cisplatin-induced nephrotoxicity and could be used for preventing renal damage caused by chemotherapeutics in cancer patients during chemotherapy.

### Example 4: Aminothiol compound protection from radiation

**The following example demonstrated** the protective effect of **the aminothiol compound of the present invention** on radiation intestinal injury, radiation lung injury, and radiation-induced hematopoietic system injury.

### 1. Protective effect on radiation intestinal injury

C57 male mice were randomly divided into 4 groups, namely a blank control group, an irradiation group, a compound B (TFA) irradiation group, and an AMI irradiation group, respectively. The mode of administration for each group was an intraperitoneal injection, 30 min before irradiation. The dosage of administration was 517 mpk, and the abdominal irradiation dose was 16 Gy. On the fifth day, the small intestine tissue was taken for HE staining.

Figure 11 showed that the normal unirradiated intestinal villi structure was clear and dense; the small intestinal villi structure changed after irradiation; and the changes in the intestinal villi structure in the compound B (TFA) irradiation group and the amifostine irradiation group were milder than that in the irradiation group, indicating that compound B (TFA) had a good protective effect on radiation intestinal injury, and the effect was similar to that of amifostine.

### 2. Protective effect on radiation lung injury

C57 male mice were randomly divided into 4 groups, namely a blank control group, an irradiation group, a compound B (TFA) irradiation group, and an AMI irradiation group, respectively. The mode of administration for each group was an intraperitoneal injection, 30 min before irradiation. The dosage of administration was 517 mpk, and the irradiation dose to the right lung was 17 Gy. On the fifth day, the small intestine tissue was taken for HE staining. Lung tissues were taken at months 2, 4, and 6 for HE staining.

Figure 12 showed that after lateral lung irradiation, the main manifestation of the lung was radiation pneumonia, and the lung tissue of the mice in the control group showed an obvious vacuole-like structure; the structure of the alveoli of the mice in the 17Gy group was changed, manifested as a large area of inflammatory infiltration in the lungs (the small blue dots were inflammatory cells), and the alveoli were filled with inflammatory cells; both amifostine and compound B could partially relieve the inflammatory cell infiltration and reduce the occurrence of radiation pneumonitis, indicating that compound B had a good protective effect on radiation lung injury, and the effect was similar to that of amifostine.

### 3. Protective effect on radiation-induced hematopoietic system injury

C57 male mice were randomly divided into 6 groups, namely a blank control group, an irradiation group, a compound B (TFA) group, an AMI group, a compound B (TFA) irradiation group, and an AMI irradiation group, respectively. The mode of administration for each group was an intraperitoneal injection, 30 min before irradiation. The dosage of administration was 517 mpk, and the whole body irradiation dose was 4 Gy. On day 15, samples were taken for detecting peripheral blood indicators.

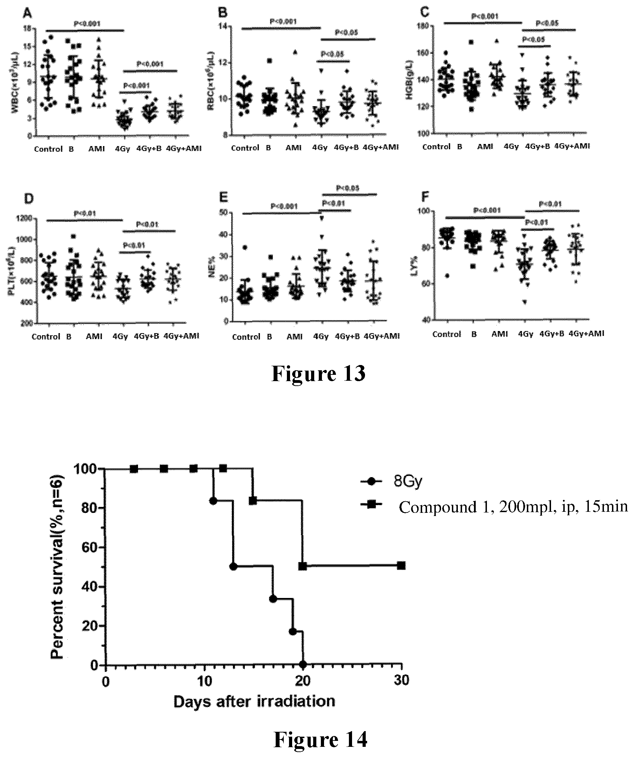

Figure 13 showed that irradiation resulted in reduced numbers of WBC, RBC, and PLT in the peripheral blood of the mice, and giving compound B (TFA) could increase the numbers of such cells after irradiation. In addition, after irradiation, the LY% decreased and the NE% increased in the peripheral blood of the mice. Compound B (TFA) could alleviate this abnormal differentiation. Therefore, compound B (TFA) had a good protective effect on the blood system.

### Example 5: Synthesis of some novel aminothiol compounds

Example 5A: Synthesis of (R)-2-(((R)-3-mercapto-2-(methylamino)propyl)amino)-3-(methylamino)propane-1-thiol hydrochloride:

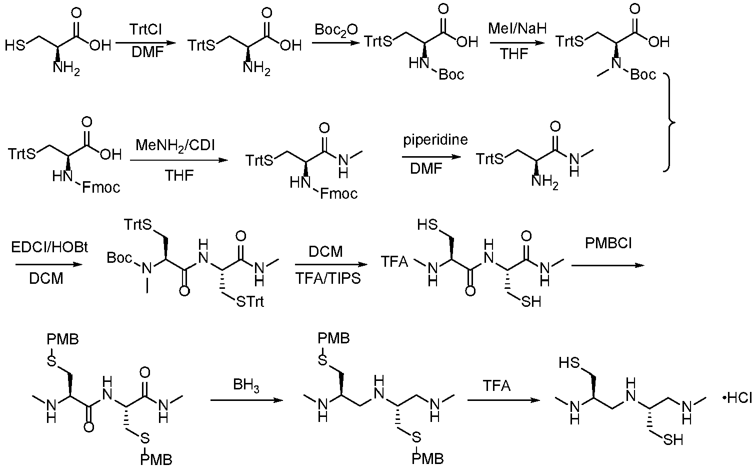

### Step 1: Synthesis of S-trityl-L-cysteine:

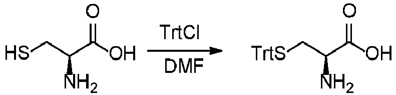

The compound L-cysteine hydrochloride (10 g, 63.45 mmol) was dissolved in N,N-dimethylformamide (120 ml), triphenylchloromethane (19.46 g, 69.795 mmol) was added, and the mixture was heated to 60-65°C and reacted for 8 hours. After TLC detected that the reaction was complete, the reaction product was cooled to room temperature, a 10% sodium acetate solution (300 ml) was added. A white solid precipitated out and was filtered, and the filter residue was washed with pure water (300 ml) and then with acetone (200 ml), and dried. The product was a white solid (17.56 g, yield: 76.15%). ¹H NMR (400MHz, DMSO-d6) δ 7.28 (m, 18H), 2.92 (dd, 1H), 2.59 (dd, 1H), 2.41 (dd, 1H).

### Step 2: Synthesis of N-(tert-butoxycarbonyl)-S-trityl-L-cysteine:

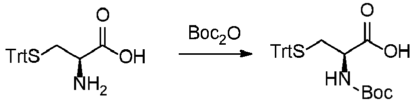

The compound S-trityl-L-cysteine (5 g, 13.76 mmol) was dissolved in a mixed solution of dioxane (40 ml), water (20 ml), and a 1M sodium hydroxide solution (14 ml), the mixture was stirred in an ice bath, Boc-anhydride (3.5 ml, 15.14 mmol) was added, and the reaction naturally rose to room temperature and was stirred for 8 hours. After TLC detected that the reaction was complete, the reaction mixture was concentrated to 20-25 ml, ethyl acetate was added, a sodium bisulfate solution was dropwise added while stirring in an ice bath, the pH was adjusted to 2-3, the reaction mixture was then extracted with ethyl acetate, and the organic layer was washed with saturated brine, then dried over sodium sulfate, concentrated, and separated by column chromatography (methanol : dichloromethane 1-5%). The product was a white solid (5.5 g, yield: 86.21%). ¹H NMR (400 MHz, DMSO-d6) δ 7.26 (m, 16H), 3.78 (d, 1H), 2.51 (m, 1H), 2.36 (dd, 1H), 1.4 (d, 9H).

### Step 3: Synthesis of N-(tert-butoxycarbonyl)-N-methyl-S-trityl-L-cysteine:

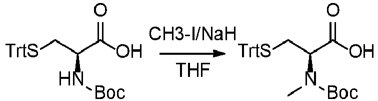

The compound N-(tert-butoxycarbonyl)-S-trityl-L-cysteine (2.1 g, 4.53 mmol) was dissolved in anhydrous tetrahydrofuran (6 ml), sodium hydride (436 mg, 10.9 mmol) was dissolved in anhydrous tetrahydrofuran (14 ml), a solution of an amino acid in tetrahydrofuran was dropwise added to a solution of sodium hydride in tetrahydrofuran under ice bath conditions, methyl iodide (0.93 ml, 14.95 mmol) was then slowly dropwise added, and the mixture was stirred overnight. After TLC detected that the reaction was complete, a phosphate buffer with pH = 7 was added for quenching, the pH was adjusted to 6-7 with a saturated ammonium chloride solution, the mixture was extracted with ethyl acetate, and the organic layer was washed with saturated brine, then dried over sodium sulfate, concentrated, and separated by column chromatography (methanol : dichloromethane 1-5%). The product obtained was a white solid (1.3 g, yield: 60.19%). ¹H NMR (400 MHz, DMSO-d6) δ 7.3 (m, 15H), 3.75 (s, 1H), 2.8 (s, 1H), 2.66 (d, 4H), 1.4 (d, 9H).

### Step 4: Synthesis of (9H-fluoren-9-yl)methyl (R)-(1-(methylamino)-1-oxo-3-(tritylthio)prop-2-yl)carbamate:

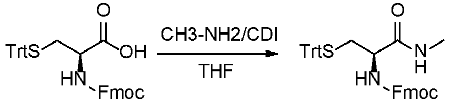

The compound (R)-2-((((9H-fluoren-9-yl)methoxy)carbonyl)amino)-3-(tritylthio)propionic acid (10 g, 17.07 mmol) was dissolved in tetrahydrofuran (50 ml), N',N-carbonyldiimidazole (5.59 g, 34.48 mmol) was added at 0-5°C, the mixture was stirred for 2 hours under nitrogen condition, methylamine (3.03 ml, 68.28 mmol) was then added, and a reaction was carried out at 0-5°C for 2 hours. After the reactants were completely consumed, 2M hydrochloric acid (60 ml) was added for quenching, the reaction mixture was extracted with dichloromethane, the organic layer was washed with saturated brine, then dried over sodium sulfate, and concentrated to obtain a crude product, and methanol (20 ml) was added and stirred at room temperature overnight. A white solid precipitated out and was filtered, and the filter residue was the product. The methanol phase was concentrated and separated by column chromatography (methanol : dichloromethane 1-5%) to obtain a white solid (9.44 g, yield: 92.37%). ¹H NMR (400 MHz, DMSO) δ 7.89 (d, 2H), 7.81 (d, 1H), 7.74 (d, 2H), 7.66 (d, 1H), 7.41 (t, 2H), 7.29 (m, 17H), 4.31 (d, 1H), 4.22 (t, 2H), 4.00 (d, 1H), 2.53 (d, 3H), 2.39 (d, 2H).

### Step 5: Synthesis of (R)-2-amino-N-methyl-3-(tritylthio)propionamide

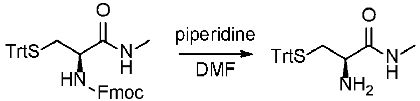

The compound (9H-fluoren-9-yl)methyl (R)-(1-(methylamino)-1-oxo-3-(tritylthio)prop-2-yl)carbamate (2 g, 3.34 mmol) was dissolved in N,N-dimethylformamide (20 ml), piperidine (0.07 ml, 0.668 mmol) was added, and the mixture was reacted at room temperature for 4 hours. After TLC detected that the reaction was complete, the reaction mixture was washed with saturated brine and extracted with dichloromethane, and the organic phase was dried over sodium sulfate, then concentrated, and separated by column chromatography (methanol : dichloromethane 1-5%). The product was a yellowish white solid (879 mg, yield: 69.76%). ¹H NMR (400 MHz, CDCl₃) δ 7.77 (d, 1H), 7.29 (m, 15H), 3.08 (m, 1H), 2.55 (d. 3H), 2.37 (dd, 1H), 2.19 (dd, 1H), 1.80 (s, 2H).

### Step 6: Synthesis of tert-butyl methyl ((R)-1-(((R)-1-(methylamino)-1-oxo-3-(tritylthio)prop-2-yl)amino)-1-oxo-3-(tritylthio)prop-2-yl)carbamic acid:

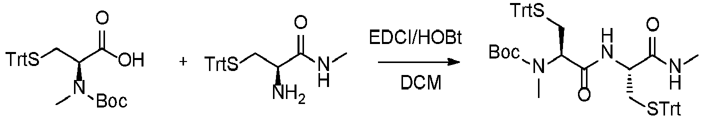

The compound N-(tert-butoxycarbonyl)-N-methyl-S-trityl-L-cysteine (150 mg, 0.314 mmol) was dissolved in dichloromethane (5 ml), 1-hydroxybenzotriazole (63.7 mg, 0.471 mmol) and EDCI (90.3 mg, 0.471 mmol) were added, the mixture was stirred at room temperature for 5 minutes, (R)-2-amino-N-methyl-3-(tritylthio)propionamide (141.9 mg, 0.377 mmol) was added, and the mixture was stirred at room temperature for 30 minutes. After TLC detected that the reaction was complete, the mixture was washed with saturated brine and extracted with dichloromethane, and the organic phase was dried over sodium sulfate, then concentrated, and purified by TLC(dichloromethane : methanol 15 : 1). The product was a white solid (260 mg, yield: 99.2%). ¹H NMR (400 MHz, CDCl₃) δ 7.39 (m, 12H), 7.22 (m, 20H), 4.1 (d, 1H), 3.95 (s, 1H), 2.61 (dd, 10H), 1.39 (s, 9H).

### Step 7: Synthesis of (R)-3-mercapto-N-((R)-3-mercapto-1-(methylamino)-1-phenylprop-2-yl)-2-(methylamino)propionamide trifluoroacetate:

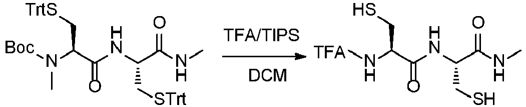

The compound tert-butyl methyl ((R)-1-(((R)-1-(methylamino)-1-oxo-3-(tritylthio)prop-2-yl)amino)-1-oxo-3-(tritylthio)prop-2-yl)carbamic acid (1.1 g, 1.32 mmol) was dissolved in dichloromethane : trifluoroacetic acid : triisopropylsilane (volume ratio 50:47:3) (25 ml), and the mixture was stirred at room temperature for 5 min. After TLC detected that the reaction was complete, the reaction product was concentrated, diethyl ether was added, the mixture was stirred in an ice bath, a white solid precipitated out, and the white solid was filtered and dried to obtain the product (400 mg, yield: 86.9%). ¹H NMR (400 MHz, MeOD) δ 4.5 (m, 1H), 4.07 (t, 1H), 3.18-2.96 (m, 3H), 2.82 (m, 1H), 2.78 (s, 3H), 2.74 (s, 3H); ESI-MS:252 [M +H]⁺.

### Step 8: Synthesis of (R)-3-((4-methoxybenzyl)thio)-N-((R)-3-((4-methoxybenzyl)thio)-1-(methylamino)-1-oxopropane-2-yl)-2-(methylamino)propionamide:

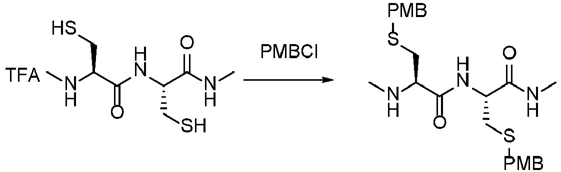

(R)-3-mercapto-N-((R)-3-mercapto-1-(methylamino)-1-phenylprop-2-yl)-2-(methylamino)propionamide trifluoroacetate (2.0 g, 5.7 mmol) was added to dichloromethane (20 mL), triethylamine (2.0 g, 20 mmol) and p-methoxybenzyl chloride (1.9 g, 12 mmol) was then added, and the mixture was stirred at 25°C for 12 hours. The reaction solution was concentrated and separated by column chromatography (methanol : dichloromethane 5-1%) to obtain a colorless oil (790 mg, yield: 29.3%). ¹H NMR (400 MHz,CDCl₃) δ 7.9 (m, 1H), 7.28-7.25 (m, 4H), 6.88-6.84 (m, 4H), 6.35 (m, 1H), 4.47-4.42 (m, 1H), 3.81 (d, 6H), 3.72-3.70 (m, 3H), 3.51 (s, 2H), 3.04-3.01 (m, 1H), 2.95-2.90 (m, 1H), 2.84-2.72 (m, 5H), 2.67-2.64 (m, 1H), 2.32 (s, 3H).

### Step 9: Synthesis of (R)-3-((4-methoxybenzyl)thio)-N²-((R)-3-((4-methoxybenzyl)thio)-2-(methylamino)propyl)-N¹-methylpropane-1,2-diamine:

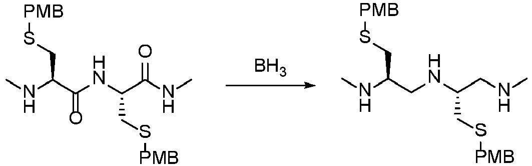

(R)-3-((4-methoxybenzyl)thio)-N-((R)-3-((4-methoxybenzyl)thio)-1-(methylamino)-1-oxopropane-2-yl)-2-(methylamino)propionamide (790 mg, 1.6 mmol) was added to tetrahydrofuran (10 mL), a borane tetrahydrofuran solution (21 mL, 21 mmol) was then added, and the mixture was heated to reflux under nitrogen protection and stirred for 8 hours. After TLC detected that the reaction was complete, the reaction product was cooled and then quenched by adding methanol (5 mL), and the reaction solution was stirred and refluxed for 1 hour. The reaction solution was concentrated to obtain a yellow oil (740 mg, yield: 100%). ¹H NMR (400 MHz, CDCl₃) δ 7.28-7.23 (m, 4H), 6.87-6.84 (m, 4H), 3.81 (s, 6H), 3.75 (t, 3H), 3.70 (s, 4H), 2.65-2.40 (m, 10H), 2.30 (dd, 3H).

### Step 10: Synthesis of (R)-2-(((R)-3-mercapto-2-(methylamino)propyl)amino)-3-(methyla mino)propane-1-thiol hydrochloride:

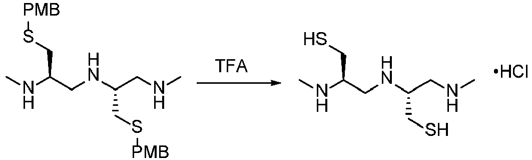

(R)-3-((4-methoxybenzyl)thio)-N²-((R)-3-((4-methoxybenzyl)thio)-2-(methylamino)propyl)-N¹-methylpropane-1,2-diamine (800 mg, 1.7 mmol) and o-cresol (1.9 g, 17 mmol) were added to trifluoroacetic acid (30 mL), and the mixture was heated to reflux under nitrogen protection and stirred for 8 hours. After TLC detected that the reaction was complete, the reaction solution was cooled and then concentrated, and water (10 mL) and diethyl ether (20 mL) were added. After liquid separation, the aqueous phase was washed with diethyl ether (15 mL). The aqueous phase was adjusted to a pH of 10 with aqueous potassium carbonate (10%) and then extracted with dichloromethane (10 mL × 2), the organic phases were combined, and a hydrogen chloride/dichloromethane solution (1 M, 10 mL) was added. After filtration, a white solid (200 mg, yield: 45%) was obtained. ¹H NMR (400 MHz, D₂O) δ 3.93 (bs, 1H), 3.71-3.67 (m, 1H), 3.44-3.35 (m, 2H), 3.29-3.22 (m, 3H), 3.17 (d, 2H), 3.03-2.99 (m, 1H), 2.67 (d, 6H); ESI-MS: 224 [M +H]⁺.

### Example 5B: Synthesis of (R)-2-(((R)-3-mercapto-2-(((R)-3-mercapto-2-(methylamino)propyl)amino)propyl)amino)-3-(methylamino)propane-1-thiol hydrochloride:

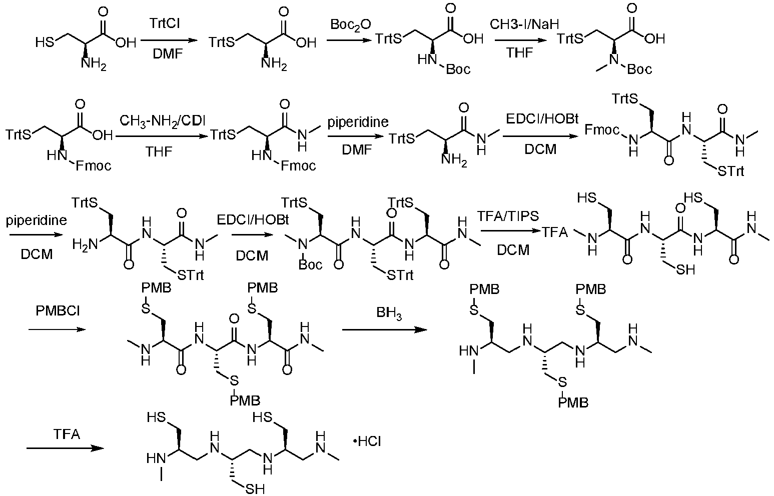

### Step 1: Synthesis of S-trityl-L-cysteine:

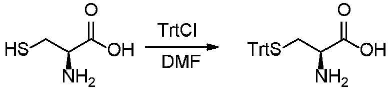

The compound L-cysteine hydrochloride (10 g, 63.45 mmol) was dissolved in N,N-dimethylformamide (120 ml), triphenylchloromethane (19.46 g, 69.795 mmol) was added, and the mixture was heated to 60-65°C and reacted for 8 hours. After TLC detected that the reaction was complete, the reaction product was cooled to room temperature, a 10% sodium acetate solution (300 ml) was added. A white solid precipitated out and was filtered, and the filter residue was washed with pure water (300 ml) and then with acetone (200 ml), and dried. The product was a white solid (17.56 g, yield: 76.15%). ¹H NMR (400 MHz, DMSO) δ 7.28 (m, 18H), 2.92 (dd, 1H), 2.59 (dd, 1H), 2.41 (dd, 1H).

### Step 2: Synthesis of N-(tert-butoxycarbonyl)-S-trityl-L-cysteine:

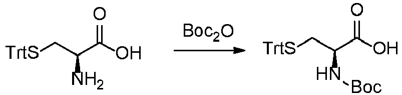

The compound S-trityl-L-cysteine (5 g, 13.76 mmol) was dissolved in a mixed solution of dioxane (40 ml), water (20 ml), and a 1M sodium hydroxide solution (14 ml), the mixture was stirred in an ice bath, Boc-anhydride (3.5 ml, 15.14 mmol) was added, and the reaction naturally rose to room temperature and was stirred for 8 hours. After TLC detected that the reaction was complete, the reaction mixture was concentrated to 20-25 ml, ethyl acetate was added, a sodium bisulfate solution was dropwise added while stirring in an ice bath, the pH was adjusted to 2-3, the reaction mixture was then extracted with ethyl acetate, and the organic layer was washed with saturated brine, then dried over sodium sulfate, concentrated, and separated by column chromatography (methanol : dichloromethane 1-5%). The product was a white solid (5.5 g, yield: 86.21%). ¹H NMR (400 MHz, DMSO-d6) δ 7.26 (m, 16H), 3.78 (m, 1H), 2.51 (m, 1H), 2.36 (dd, 1H), 1.4 (s, 9H).

### Step 3: Synthesis of N-(tert-butoxycarbonyl)-N-methyl-S-trityl-L-cysteine:

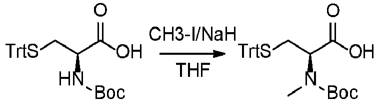

The compound N-(tert-butoxycarbonyl)-S-trityl-L-cysteine (2.1 g, 4.53 mmol) was dissolved in anhydrous tetrahydrofuran (6 ml), sodium hydride (436 mg, 10.9 mmol) was dissolved in anhydrous tetrahydrofuran (14 ml), a solution of an amino acid in tetrahydrofuran was dropwise added to a solution of sodium hydride in tetrahydrofuran under ice bath conditions, methyl iodide (0.93 ml, 14.95 mmol) was then slowly dropwise added, and the mixture was stirred overnight. After TLC detected that the reaction was complete, a phosphate buffer with pH = 7 was added for quenching, the pH was adjusted to 6-7 with a saturated ammonium chloride solution, the mixture was extracted with ethyl acetate, and the organic layer was washed with saturated brine, then dried over sodium sulfate, concentrated, and separated by column chromatography (methanol : dichloromethane 1-5%), The product was a white solid (1.3 g, yield: 60.19%). ¹H NMR (400 MHz, DMSO-d6) δ 7.3 (m, 15H), 3.75 (s, 1H), 2.8 (s, 1H), 2.66 (d, 4H), 1.4 (d, 9H).

### Step 4: Synthesis of (9H-fluoren-9-yl)methyl (R)-(1-(methylamino)-1-oxo-3-(tritylthio)prop-2-yl)carbamate:

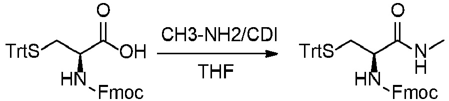

The compound (R)-2-((((9H-fluoren-9-yl)methoxy)carbonyl)amino)-3-(tritylthio)propionic acid (10 g, 17.07 mmol) was dissolved in tetrahydrofuran (50 ml), N',N-carbonyldiimidazole (5.59 g, 34.48 mmol) was added at 0-5°C, the mixture was stirred for 2 hours under nitrogen condition, methylamine (3.03 ml, 68.28 mmol) was then added, and a reaction was carried out at 0-5°C for 2 hours. After the reactants were completely consumed, 2M hydrochloric acid (60 ml) was added for quenching, the reaction mixture was extracted with dichloromethane, the organic layer was washed with saturated brine, then dried over sodium sulfate, and then concentrated to obtain a crude product, and methanol (20 ml) was added and stirred at room temperature overnight. A white solid precipitated out and was filtered, and the filter residue was the product. The methanol phase was concentrated and separated by column chromatography (methanol : dichloromethane 1-5%) to obtain a white solid (9.44 g, yield: 92.37%). ¹H NMR (400 MHz, DMSO-d6) δ 7.89 (d, 2H), 7.81 (d, 1H), 7.74 (d, 2H), 7.66 (d, 1H), 7.41 (t, 2H), 7.29 (m, 17H), 4.31 (d, 1H), 4.22 (t, 2H), 4.00 (d, 1H), 2.53 (d, 3H), 2.39 (d, 2H).

### Step 5: Synthesis of (R)-2-amino-N-methyl-3-(tritylthio)propionamide:

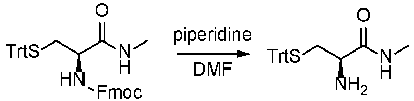

The compound (9H-fluoren-9-yl)methyl (R)-(1-(methylamino)-1-oxo-3-(tritylthio)prop-2-yl)carbamate (2 g, 3.34 mmol) was dissolved in N,N-dimethylformamide (20 ml), piperidine (0.07 ml, 0.668 mmol) was added, and the mixture was reacted at room temperature for 4 hours. After TLC detected that the reaction was complete, the reaction mixture was washed with saturated brine and extracted with dichloromethane, and the organic phase was dried over sodium sulfate, then concentrated, and separated by column chromatography (methanol : dichloromethane 1-5%). The product was a yellowish white solid (879 mg, yield: 69.76%). ¹H NMR (400 MHz, DMSO-d6) δ 7.77 (d, 1H), 7.29 (m, 15H), 3.08 (m, 1H), 2.55 (d, 3H), 2.37 (m, 1H), 2.19 (m, 1H), 1.80 (s, 2H).

### Step 6: Synthesis of (9H-fluoren-9-yl)methyl ((R)-1-(((R)-1-(methylamino)-1-oxo-3-(tritylthio)prop-2-yl)amino)-1-oxo-3-(tritylthio)prop-2-yl)carbamic acid:

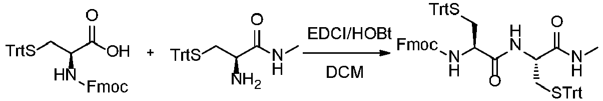

The compound (R)-2-((((9H-fluoren-9-yl)methoxy)carbonyl)amino)-3-(tritylthio)propionic acid (100 mg, 0.17 mmol) was dissolved in dichloromethane (5 ml), 1-hydroxybenzotriazole (34.5 mg, 0.255 mmol) and EDCI (48.9 mg, 0.255 mmol) were added, the mixture was stirred at room temperature for 5 minutes, (R)-2-amino-N-methyl-3-(tritylthio)propionamide (76.8 mg, 0.204 mmol) was added, and the mixture was reacted at room temperature for 30 minutes. After TLC detected that the reaction was complete, the mixture was washed with saturated brine and extracted with dichloromethane, and the organic phase was dried over sodium sulfate, then concentrated, and separated by column chromatography (methanol : dichloromethane 1-5%) to obtain a white solid (160 mg, yield: 99.68%) as the product. ¹H NMR (400 MHz, DMSO-d6) δ 7.89 (d, 2H), 7.71 (m, 4H), 7.40 (m, 2H), 7.38-7.25 (m, 30H), 4.25 (m, 4H), 4.01 (m, 1H), 2.50-2.33 (m, 7H).

### Step 7: Synthesis of (R)-2-amino-N-((R)-1-(methylamino)-1-oxo-3-(tritylthio)prop-2-yl)-3-(tritylthio)propionamide:

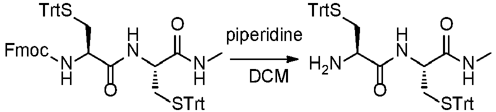

The compound (9H-fluoren-9-yl)methyl ((R)-1-(((R)-1-(methylamino)-1-oxo-3-(tritylthio)prop-2-yl)amino)-1-oxo-3-(tritylthio)prop-2-yl)carbamic acid (3.6 g, 3.8 mmol) was dissolved in N,N-dimethylformamide (15 ml), piperidine (0.07 ml, 0.76 mmol) was added, and the mixture was reacted at room temperature for 4 hours. After TLC detected that the reaction was complete, the reaction mixture was washed with saturated brine and extracted with dichloromethane, and the organic phase was dried over sodium sulfate, then concentrated, and separated by column chromatography (methanol : dichloromethane 1-5%), The product was a white solid (1.3 g, yield: 47.44%). ¹H NMR (400 MHz, DMSO-d6) δ 8.12 (s, 1H), 7.83 (d, 1H), 7.27 (m, 30H), 4.25 (s, 1H), 3.29 (m, 2H), 3.20 (s, 1H), 2.65-2.23 (m, 5H).

### Step 8: Synthesis of tert-butyl methyl((4R,7R,10R)-3,6,9-trioxo-13,13,13-triphenyl-4,7-bis((tritylthio)methyl)-12-thia-2,5-8-triazatridecan-10-yl)carbamate:

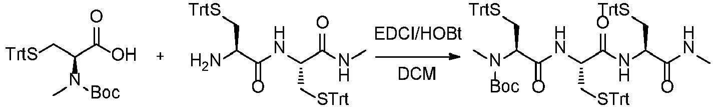

The compound N-(tert-butoxycarbonyl)-N-methyl-S-trityl-L-cysteine (509 mg, 1.07 mmol) was dissolved in dichloromethane (10 ml), 1-hydroxybenzotriazole (218 mg, 1.61 mmol) and EDCI (309 mg, 1.61 mmol) were added, the mixture was stirred at room temperature for 5 minutes, (R)-2-amino-N-((R)-1-(methylamino)-1-oxo-3-(tritylthio)prop-2-yl)-3-(tritylthio)propionamide (924 mg, 1.28 mmol) was added, and the mixture was stirred at room temperature for 30 minutes. After TLC detected that the reaction was complete, the mixture was washed with saturated brine and extracted with dichloromethane, the organic phase was dried over sodium sulfate, concentrated, and separated by column chromatography (methanol : dichloromethane 1-5%) to obtain a white solid as the product (985 mg, yield: 77.93%). ¹H NMR (400 MHz, DMSO-d6) δ 8.11 (d, 1H), 7.73 (d, 2H), 7.32-7.21 (m, 45H), 4.25 (m, 3H), 2.62 (m, 1H), 2.49 (m, 6H) 2.47-2.23 (m, 5H), 1.35-1.21 (d, 9H).

### Step 9: Synthesis of (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide trifluoroacetate:

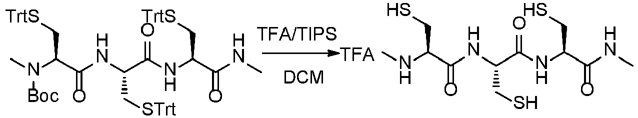

Tert-butyl methyl((4R,7R,10R)-3,6,9-trioxo-13,13,13-triphenyl-4,7-bis((tritylthio)methyl)-12-thia-2,5-8-triazatridecan-10-yl)carbamate (1.5 g, 1.27 mmol) was dissolved in dichloromethane : trifluoroacetic acid : triisopropylsilane (volume ratio 50:47:3) (40 ml), and the mixture was stirred at room temperature for 5 min. After TLC detected that the reaction was complete, the reaction product was concentrated, diethyl ether was added, the mixture was stirred in an ice bath, a white solid precipitated out and was filtered and dried to obtain the product (446 mg, yield: 77.77%). ¹HNMR(400 MHz, MeOD)δ 4.54 (m, 1H), 4.42 (m, 1H), 4.05 (t, 1H), 3.13 (m, 1H),2.99 (m, 2H), 2.85 (m, 2H), 2.79 (m, 1H), 2.73 (s, 3H), 2.70 (s, 3H). ESI-MS: 355, [M+H]⁺.

### Step 10: Synthesis of (R)-3-((4-methoxybenzyl)thio)-N-((R)-3-((4-methoxybenzyl)thio)-1-(((R)-3-((4-methoxybenzyl)thio)-1-(methylamino)-1-oxopropane-2-yl)amino)-1-oxopropane-2-yl)-2-(methylamino)propionamide:

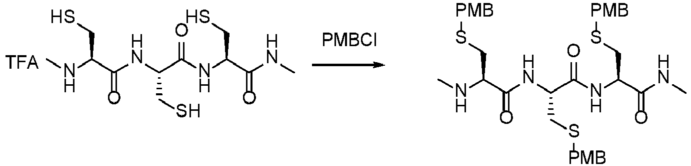

(R)-3-mercapto-N-((R)-3-mercapto-1-(((R)-3-mercapto-1-(methylamino)-1-oxoprop-2-yl)amino)-1-oxoprop-2-yl)-2-(methylamino)propionamide trifluoroacetate (1.1 g, 2.4 mmol) was added to dichloromethane (20 mL), triethylamine (1.1 g, 11 mmol) and p-methoxybenzyl chloride (1.1 g, 7.2 mmol) were then added, and the mixture was stirred at 25°C for 12 hours. The reaction solution was concentrated and separated by column chromatography (methanol : dichloromethane 5-1%) to obtain a colorless oil (640mg, yield: 36%). ¹H NMR (400 MHz, CDCl₃) δ 7.84 (s, 1H), 7.28-7.22 (m, 6H), 6.96 (d, 1H), 6.88-6.83 (m, 6H), 6.70 (s, 1H) 4.55-4.53 (m, 2H),3.79 (s, 9H), 3.71-3.67 (m, 7H), 3.03-2.79 (m, 6H), 2.80 (d, 3H), 2.65-2.61 (m, 1H) 2.34 (s, 3H).

### Step 11: Synthesis of (R)-3-((4-methoxybenzyl)thio)-N²-((R)-3-((4-methoxybenzyl)thio)-2-(((R)-3-((4-methoxybenzyl)thio)-2-(methylamino)propyl)amino)propyl)-N¹methylpropane-1,2-diamine:

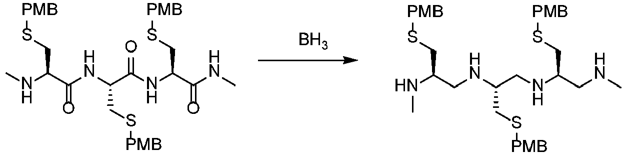

(R)-3-((4-methoxybenzyl)thio)-N-((R)-3-((4-methoxybenzyl)thio)-1-(((R)-3-((4-methoxybenzyl)thio)-1-(methylamino)-1-oxopropane-2-yl)amino)-1-oxopropane-2-yl)-2-(methylamino)propionamide (3.2 g, 4.5 mmol) was added to tetrahydrofuran (100 mL), borane tetrahydrofuran solution (140 mL, 140 mmol) was then added, and the mixture was heated to reflux under nitrogen protection and stirred for 8 hours. After TLC detected that the reaction was complete, the reaction product was cooled and then quenched by adding methanol (20 mL), and the reaction solution was stirred and refluxed for 1 hour. The reaction solution was concentrated to obtain a yellow oil (3.1g, yield: 100%). ¹H NMR (400 MHz, CDCl₃) δ 7.25-7.22 (m, 6H), 6.86-6.84 (m, 6H), 3.80 (d, 9H), 3.67 (d, 6H), 2.65-2.44 (m, 19H), 2.30 (d, 6H).

### Step 12: Synthesis of (R)-2-(((R)-3-mercapto-2-(((R)-3-mercapto-2-(methylamino)propyl)amino)propyl)amino)-3-(methylamino)propane-1-thiol hydrochloride:

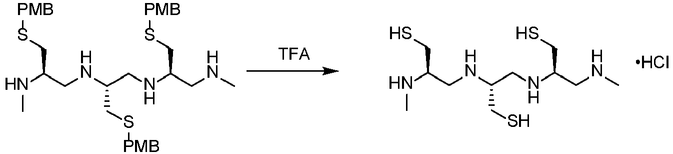

(R)-3-((4-methoxybenzyl)thio)-N²-((R)-3-((4-methoxybenzyl)thio)-2-(methylamino)propyl)-N¹-methylpropane-1,2-diamine (3.1 g, 4.5 mmol) and o-cresol (15 g, 140 mmol) were added to trifluoroacetic acid (120 mL), and the mixture was heated to reflux under nitrogen protection and stirred for 8 hours. After TLC detected that the reaction was complete, the reaction solution was cooled and then concentrated, and water (15 mL) and diethyl ether (30 mL) were added. After liquid separation, the aqueous phase was washed with diethyl ether (30 mL). The aqueous phase was then adjusted to a pH of 10 with aqueous potassium carbonate (10%) and extracted with dichloromethane (10 mL × 2), the organic phases were combined, and a hydrogen chloride/dichloromethane solution (1 M, 30 mL) was added. After filtration, a white solid (200 mg, yield: 28%) was obtained. ¹H NMR (400 MHz, D₂O) δ 4.01-3.93 (m, 1H), 3.75-3.70 (m, 1H), 3.63-3.61 (m, 1H), 3.44-3.37 (m, 3H), 3.27-2.93 (m, 10H), 2.70 (d, 3H), 2.65 (d, 3H); ESI-MS: 313 [M +H]⁺.

### Example 5C: Synthesis of (R)-N-((R)-3-((2-methoxyprop-2-yl)thio)-1-(methylamino)-1-oxopropane-2-yl)-2,2,3-trimethylthiazolidine-4-carboxamide:

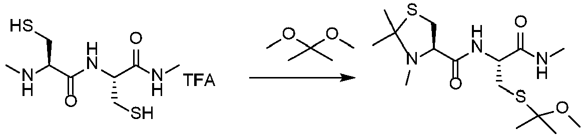

(R)-3-mercapto-N-((R)-3-mercapto-1-(methylamino)-1-phenylprop-2-yl)-2-(methylamino)propionamide trifluoroacetate (4 g, 11 mmol), 1 g of montmorillonite K10, 50 ml of acetone, and 150 ml 2,2-dimethoxypropane were mixed, dissolved and stirred at 25°C under N₂ protection for 24 h. After TLC detected that the reaction was complete, the reaction solution was filtered, and the filtrate was spin-dried and separated by column chromatography (neutral Al₂O₃, MeOH/DCM = 1/450) to obtain 600 mg of the product, with a yield of 15.1%. ¹H NMR (400 MHz, CDCl₃) δ 7.86 (d, 1H), 6.29 (s, 1H), 4.49 (dd, 1H), 3.53 (dd, 1H), 3.41 (dd, 1H), 3.30 (s, 3H), 3.10 (dd, 1H), 3.00-2.77 (m, 5H), 2.34 (s, 3H), 1.65-1.53 (m, 9H), 1.46 (s, 3H), ESI-MS: 365 [M +H]⁺.

### Example 5D: Synthesis of (R)-2,2,3-trimethyl-N-((6R, 9R)-3,3,12,12-tetramethyl-6-(methylcarbamoyl)-8-oxo-2,13-dioxa-4,11-dithia-7-azatetradecan-9-yl)thiazolidine-4-carboxamide:

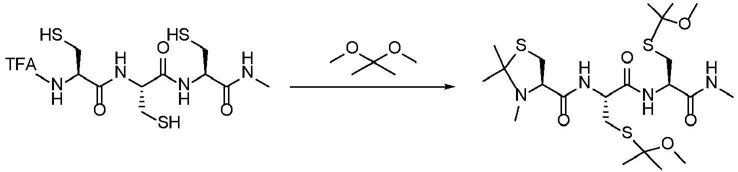

(R)-3-mercapto-N-((R)-3-mercapto-1-(((R)-3-mercapto-1-(methylamino)-1-oxoprop-2-yl)amino)-1-oxoprop-2-yl)-2-(methylamino)propionamide trifluoroacetate (4.5 g, 9.6 mmol), 0.9 g of montmorillonite K10, 45 ml of acetone, and 135 ml of 2,2-dimethoxypropane were mixed, dissolved and stirred at 25°C under nitrogen protection for 24 h. After TLC detected that the reaction was complete, the reaction solution was filtered, and the filtrate was spin-dried and separated by column chromatography (neutral Al₂O₃, MeOH/DCM = 1/350) to obtain 650 mg of the product, with a yield of 12.5%. ¹H NMR (400 MHz, CDCl₃) δ 7.95 (d, 1H), 7.36 (d, 1H), 6.78 (s, 1H), 4.68 (dt, 1H), 4.56 (dd, 7.3 Hz, 1H), 3.57 (dd, 1H), 3.42 (dd, 1H), 3.32 (d, 6H), 3.15 (ddd, 2H), 2.93 (ddd, 3H), 2.84 (d, 3H), 2.40 (s, 3H), 1.58 (dt, 15H), 1.48 (s, 3H), ESI-MS: 539 [M +H]⁺.

### Example 5E: (R)-3-mercapto-N-((R)-3-mercapto-1-((2-(methylamino)ethyl)amino)-1-oxo propane-2-yl)-2-((2-(methylamino)ethyl)amino)propionamide

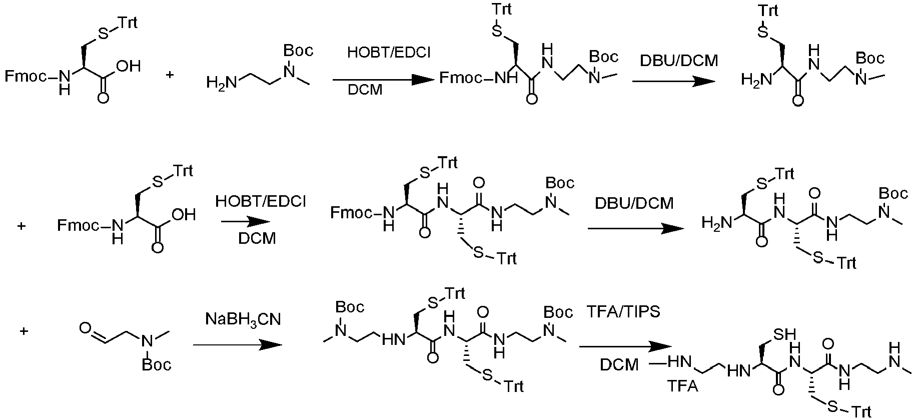

### Step 1: Synthesis of tert-butyl (R)-(2-(2-((((9H-fluoren-9-yl)methoxy)carbonyl)amino)-3-(tritylthio)propionylamino)ethyl)(methyl)carbamate

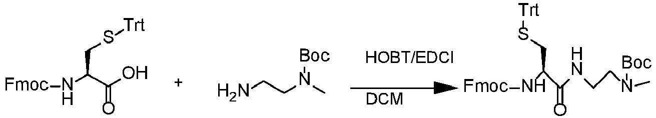

N-(((9H-fluoren-9-yl)methoxy)carbonyl)-S-trityl-L-cysteine (10 g, 17.07 mmol) was dissolved in 100 mL of dichloromethane, 1-hydroxybenzotriazole (HOBT) and 1-ethyl-(3-dimethylaminopropyl)carbodiimide hydrochloride (EDCI) were added at room temperature, the mixture was stirred for 10 minutes, tert-butyl (2-aminoethyl)(methyl)carbamate (3.6 g, 20.48 mmol) was added, and the mixture was stirred at room temperature for 40 minutes. After TLC detected that the reaction was complete, 50% saturated sodium chloride and water were added for quenching, the reaction mixture was extracted with ethyl acetate, the organic phase was washed with saturated brine, then dried over sodium sulfate, and concentrated to obtain a crude product, which was separated by chromatography (petroleum ether : ethyl acetate = 3:1-1:3) to obtain a white solid (10 g, yield 86%) as the target product, HESI: 742.45 [M +H]⁺.

### Step 2: Synthesis of tert-butyl (R)-2-(2-amino-3-(triphenylthio)propionylamino)ethyl(me thyl)carbamate

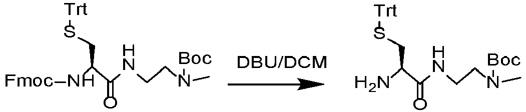

Tert-butyl (R)-(2-(2-((((9H-fluoren-9-yl)methoxy)carbonyl)amino)-3-(tritylthio)propionylamino)ethyl)(methyl)carbamate (8.8 g, 11.86 mmol) was dissolved in 80 mL of dichloromethane, 1,8-diazabicycloundec-7-ene (DBU) (8.2 g, 53.90 mmol) was added, and the mixture was stirred at room temperature for 40 minutes. After TLC detected that the reaction was complete, 50% saturated sodium chloride and water were added for quenching, the reaction mixture was extracted with ethyl acetate, and the organic phase was washed with saturated brine, then dried over sodium sulfate, and concentrated to obtain a crude product, which was separated by chromatography (petroleum ether : ethyl acetate = 3:1-1:3) to obtain a white solid (5.6 g, yield 89%) as the target product. ¹H NMR (400 MHz, chloroform-d) δ 7.49-7.44 (m, 5H), 7.34-7.28 (m, 7H), 7.26-7.21 (m, 3H), 3.33 (s, 4H), 3.04 (s, 1H), 2.85 (s, 3H), 2.77 (d, J = 12.6 Hz, 1H), 2.54 (s, 1H), 1.46 (s, 9H).

### Step 3: Synthesis of tert-butyl ((5R,8R)-1-(9H-fluoren-9-yl)-3,6,9-trioxo-5,8-bis((tritylthio)methyl)-2-oxa-4,7,10-tricystein-12-yl)(methyl)carbamate:

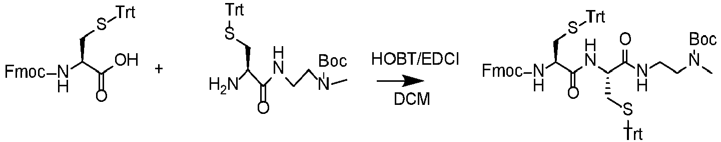

N-(((9H-fluoren-9-yl)methoxy)carbonyl)-S-trityl-L-cysteine (10 g, 17.07 mmol) was dissolved in 100 mL of dichloromethane, 1-hydroxybenzotriazole (HOBT) and 1-ethyl-(3-dimethylaminopropyl)carbodiimide hydrochloride (EDCI) were added at room temperature, the mixture was stirred for 10 minutes, tert-butyl (R)-2-(2-amino-3-(triphenylthio)propionylamino)ethyl(methyl)carbamate was added, and the mixture was then stirred at room temperature for 40 minutes. After TLC detected that the reaction was complete, 50% saturated sodium chloride and water were added for quenching, the reaction mixture was extracted with ethyl acetate, and the organic phase was washed with saturated brine, then dried over sodium sulfate, and concentrated to obtain a crude product, which was separated by chromatography (petroleum ether : ethyl acetate = 3:1-1:3) to obtain a white solid (9.8 g, yield 87.5%) as the target product, ¹H NMR (400 MHz, chloroform-d) δ 7.77 (t, J = 6.8 Hz, 2H), 7.56 (d, J = 7.4 Hz, 2H), 7.47-7.33 (m, 14H), 7.28-7.13 (m, 20H), 5.00 (d, J = 6.3 Hz, 1H), 4.38 (dt, J = 17.2, 9.2 Hz, 2H), 4.17 (dd, J = 16.8, 7.2 Hz, 2H), 3.26 (s, 2H), 3.14 (s, 2H), 2.79 (s, 3H), 2.76-2.36 (m, 4H), 1.45 (s, 9H)

### Step 4: Synthesis of tert-butyl (2-((R)-2-((R)-2-amino-3-(tritylthio)propionylamino)-3-(tritylthio)propionylamino)ethyl)(methyl)carbamate

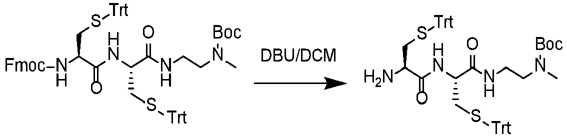

Tert-butyl ((5R,8R)-1-(9H-fluoren-9-yl)-3,6,9-trioxo-5,8-bis((tritylthio)methyl)-2-oxa-4,7, 10-tricystein-12-yl)(methyl)carbamate (2 g, 1.840 mmol) was dissolved in 50 mL of dichl oromethane, 1,8-diazabicycloundec-7-ene (DBU) (8.2 g, 53.90 mmol) was added, and the mixture was stirred at room temperature for 40 minutes. After TLC detected that the rea ction was complete, 50% saturated sodium chloride and water were added for quenching, the reaction mixture was extracted with ethyl acetate, and the organic phase was washed with saturated brine, then dried over sodium sulfate, and concentrated to obtain a crude product, which was separated by chromatography (petroleum ether : ethyl acetate = 3:1-1:3) to obtain a white solid (1.28 g, yield 80%) as the target product. ¹H NMR (400 M Hz, chloroform-d) δ 7.58-7.14 (m, 30H), 4.28-4.06 (m, 1H), 3.24 (s, 4H), 2.82 (s, 3H), 2.71 (s, 2H), 2.47 (s, 2H), 1.43 (s, 9H).

### Step 5: Synthesis of tert-butyl ((6R,9R)-7,10-dioxo-6,9-bis((tritylthio)methyl)-2,5,8,11-tetraazatridecan-13-yl)(methyl)carbamate

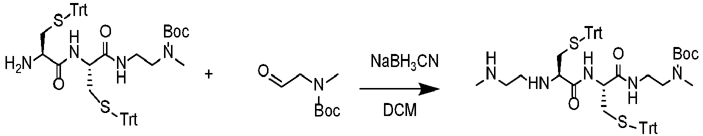

Tert-butyl (2-((R)-2-((R)-2-amino-3-(tritylthio)propionylamino)-3-(tritylthio)propionylami no)ethyl)(methyl)carbamate (500 mg, 0.578 mmol) was dissolved in 10 mL of a methano 1 solution, sodium borohydride was added at 0°C, a drop of glacial acetic acid was adde d, tert-butyl methyl(2-oxoethyl)carbamate (150 mg, 0.868 mmol) was added, the mixture was stirred at room temperature for 1 hour and concentrated in vacuo, the reaction produ ct was extracted with ethyl acetate, and the organic phase was washed with saturated bri ne, then dried over sodium sulfate, and concentrated to obtain a crude product, which w as separated by chromatography (petroleum ether : ethyl acetate = 3:1) to obtain the pro duct (370 mg, yield 63%). ¹H NMR (400 MHz, chloroform-d) δ 7.49-7.33 (m, 13H), 7.2 9-7.19 (m, 18H), 3.98 (m, 1H), 3.24-3.16 (m, 6H), 2.81 (s, 3H), 2.79 (s, 3H), 2.65-2.62 (m, 2H), 2.58-2.55 (m, 2H), 2.51-2.36 (m, 3H), 1.45 (s, 9H), 1.43 (s, 9H).

### Step 6: Synthesis of (R)-3-mercapto-N-((R)-3-mercapto-1-((2-(methylamino)ethyl)amin o)-1-oxopropane-2-yl)-2-((2-(methylamino)ethyl)amino)propionamide

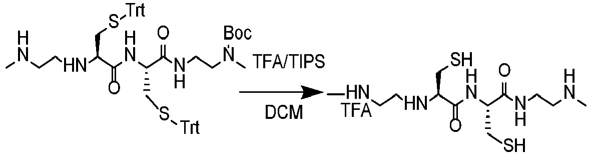

Tert-butyl ((6R,9R)-7,10-dioxo-6,9-bis((tritylthio)methyl)-2,5,8,11-tetraazatridecan-13-yl) (methyl)carbamate (360 mg, 0.390 mmol) was dissolved in 5 mL of a dichloromethane s olution, trifluoroacetic acid (4.45 g, 39.03 mmol) and triisopropylsilane (0.17 g, 0.109 m mol) were added under nitrogen protection, and the mixture was stirred for 40 minutes. After TLC detected that the reaction was complete, the reaction system was concentrated in vacuo and washed with methyl tert-butyl ether to obtain the product (TFA salt form, 125 mg, yield 81%), with an overall yield of 25.7% over the six steps of reaction, ¹H NMR (400 MHz, deuterium oxide) δ 4.48 (dd, J = 7.8, 5.3 Hz, 1H), 4.23 (s, 1H), 3.50 (q, J = 5.8 Hz, 2H), 3.45-3.29 (m, 4H), 3.17-3.11 (m, 3H), 3.04 (dd, J = 14.9, 6.0 Hz, 1H), 2.94 (dd, J = 14.3, 5.3 Hz, 1H), 2.83 (dd, J = 14.2, 7.7 Hz, 1H), 2.71 (s, 3H), 2. 66 (s, 3H). HESI: 436.70 [M+H]+

In a similar manner, the following Examples 5F, 5G and 5H were prepared.

### Example 5F: (R)-N-((7R,10R)-6,9-dioxo-13,13,13-triphenyl-7-(tritylthiomethyl)-12-thia-2,5,8-triazatridecan-10-yl)-3-mercapto-2-(2-(methylamino)ethylamino)propionamide

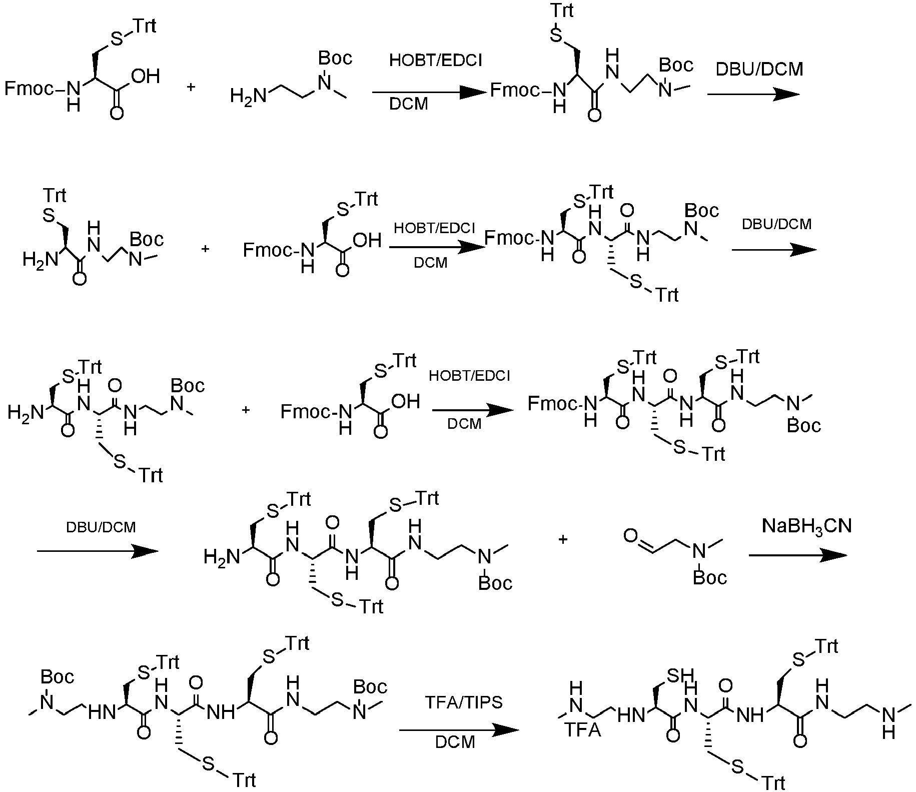

### Example 5G: (R)-N-((R)-3,3-dimethyl-7-oxo-2-oxa-4-thia-8,11-diazadodecan-6-yl)-3-(2-methoxyprop-2-ylthio)-2-(2,2,3-trimethylimidazolidin-1-yl)propionamide

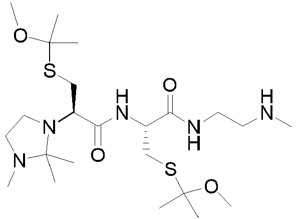

### Example 5H: (R)-N-((R)-3,3-dimethyl-7-oxo-2-oxa-4-thia-8,11-diazadodecan-6-yl)-3-(2-methoxyprop-2-ylthio)-2-((R)-3-(2-methoxyprop-2-ylthio)-2-(2,2,3-trimethylimidazolidin-1-yl)p ropionylamino)propionamide

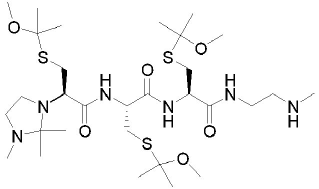

### Example 51: Examples of biological activity - Protective effects of compounds 1, 2, and 3.

Material: A γ-ray irradiation device was involved, which was a ¹³⁷Cs irradiator with a dose rate of 0.99 Gy/min. C57BL/6 male mice weighing 21-22 g were purchased from Beijing HFK Bioscience Co., LTD, with certification no. SCXK (Jing) 2014-0004, and the grouping was: an irradiation and blank solvent group and an irradiation and administration group, with 6 mice in each group. The structures of compounds 1, 2, and 3 (prepared from Examples 5A, 5C, and 5D, respectively) were shown in Table 1.

**Table 1 Nomenclature and structural formulas of compounds 1-3**

| Compound No. | Compound name | Structural formula |
|---|---|---|
| Compound 1 | (R)-2-(((R)-3-mercapto-2-(methylamino)propyl)amino)-3-(methylamino)propane-1-thiol hydrochloride | 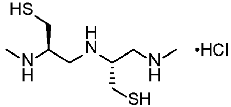 |
| Compound 2 | (R)-N-((R)-3-((2-methoxyprop-2-yl)thio)-1-(methylamino)-1-oxopropane-2-yl)-2,2,3-trimethylthiazolidine-4-carboxamide | 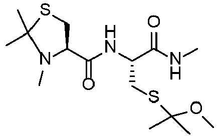 |
| Compound 3 | (R)-2,2,3-trimethyl-N-((6R,9R)-3,3,12,12-tetramethyl-6-(methylcarbamoyl)-8-oxo-2,13-dioxa-4,11-dithia-7-azatetradecan-9-yl)thiazolidine-4-carboxamide | 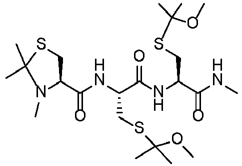 |

Method: The irradiation and drug treatment method was one time whole body irradiation with ¹³⁷Cs γ-rays, with an irradiation dose rate of 0.99 Gy/min, wherein the absorbed dose in the mice was 8.0 Gy; and compound 1 was dissolved in physiological saline, the mixture was shaken uniformly at the time of administration, and compound 1 was injected intraperitoneally (200 mg/kg BW) 30 min before irradiation. The control group was injected with physiological saline, and the irradiation dose was 8.0 Gy. The 30-day survival situation of the mice in each group was observed, and the survival rate was calculated.

Results: Intraperitoneal injection of compound 1 (200 mg/kg BW) with one time whole body irradiation by 8.0 Gy ¹³⁷Cs γ rays was compared with the irradiation and blank solvent group, and the survival rate of the mice in each group was shown in Figure 1.

Method: The irradiation and drug treatment method was one time whole body irradiation with ¹³⁷Cs γ-rays, with an irradiation dose rate of 0.99 Gy/min, wherein the absorbed dose in the mice was 8.0 Gy; and compounds 2 and 3 were dissolved in a mixed solvent of 85% HP-β-CD and 15% Solutol HS, and the mixture was shaken uniformly at the time of administration. Compound 2 was administered intragastrically (1600 mg/kg BW) 4 hours before irradiation, and compound 3 was administered intragastrically (1500 mg/kg BW) 1 hour before irradiation. The control group was given a mixed solvent of 85% HP-β-CD and 15% Solutol HS by gavage, and the irradiation dose was 8.0 Gy. The 30-day survival situation of the mice in each group was observed, and the survival rate was calculated.

Results: Intragastric administration of compound 2 (1600 mg/kg BW) with one time whole body irradiation by 8.0 Gy ¹³⁷Cs γ rays was compared with the irradiation and blank solvent group, and the survival rate of the mice in each group was shown in Figure 2. Intragastric administration of compound 3 (1500 mg/kg BW) with one time whole body irradiation by 8.0 Gy ¹³⁷Cs γ rays was compared with the irradiation and blank solvent group, and the survival rate of the mice in each group was shown in Figure 3.

### Example 6: Evaluation of the effect of the compound of the present invention in myocardial ischemia reperfusion model

The following myocardial ischemia reperfusion model was used to evaluate the effect of the compound of the present invention.

The rats were anesthetized by intraperitoneal injection with 1.0 g·kg-1 urethane solution;

Needle-shaped electrodes were inserted subcutaneously into the limbs of the rats, an electrocardiograph was connected, lead II was traced to continuously monitor the changes of the electrocardiogram, and rats with normal electrocardiogram were then selected for testing, while the abnormal ones were removed;

An incision was made in the center of the neck of the rat, the trachea was separated, and an airway tube was inserted. A small animal ventilator was connected, and it was ensured that the breathing rate thereof was 7080 breaths/minute, the inspiratory expiratory ratio was 1.5:1, and the tidal volume was 11.5 mL/100 g. Initially, the common artery of the right neck was separated and a left heart catheter was inserted to observe the various parameters of heart function; the chest was opened at the 24th intercostal space on the left edge of the sternum, the ribs were pulled away with a retractor to expose the heart, and after a 10-min wait, the blood pressure and breathing were stabilized; The left anterior descending coronary artery (LAD) was ligated with a 3-0 suture for ischemic injury and the ischemia lasted for 30 min; and after 30 min, the suture was removed, and the blood supply was restored for reperfusion injury. A TTC staining method was used to evaluate the myocardial infarction area.

Criteria for successful modeling:
Criteria for successful coronary artery ligation: When S-T segment elevation and/or tall T-wave were evident on the electrocardiogram and cyanosis occurred at the distal end of an epicardial suture, successful coronary artery ligation was indicated.
Criteria for successful post-ischemia reperfusion: When more than 50% of the elevated S-T segment decreased and the color of the epicardium returned to the original color, it was indicated that the post-ischemia reperfusion model was successfully constructed.

Experimental exclusion criteria:
Model of excessive bleeding during experimental surgery
Before ischemic treatment, the rats developed severe arrhythmia, 67%: including ventricular fibrillation, ventricular flutter, atrial fibrillation, atrial flutter, supraventricular tachycardia, ventricular tachycardia, frequent ventricular premature beat, atrioventricular block, bundle branch block, etc.
The rats experienced a continuous decrease of more than 70 mm Hg in mean arterial pressure.

### Example 7: Evaluation of the effect of the compound of the present invention in myocardial infarction model

1. The rats were grouped and administered according to the following methods
   (1) Model
   (2) Compound B (i.v.) at 100 mg/kg
   (3) Compound A (i.v.) at 100 mg/kg
   (4) Compound A (oral) at 500 mg/kg
   (5) Isosorbide mononitrate injection (i.v.) at 1 mg/kg (equivalent to clinical equivalent dose).

There were 10 rats in each group, and the rats were administered intravenously immediately after 30 min of ischemia and reperfusion, i.v. 10% 1 min + i.v. drip 90% 30 min. The administration volume was 2 ml/rat. Compound A (oral) at 500 mg/kg was administered intragastrically 2 h after reperfusion when the animal was awake, and the administration volume was 10 ml/kg.

Note: Compounds A and B used in this example were in the form of hydrochloride salts thereof.

### 2. Model preparation

Given intraperitoneal injection of 3% sodium pentobarbital at 60 mg/kg for general anesthesia, the rats were fixed on an operating table in the supine position. The surgical field of the anterolateral chest was depilated and routinely disinfected. The skin and muscular layer were cut open, purse-string suture was performed on the muscular layer, the chest was opened on the left at the fourth intercostal space, and the heart was pulled out with a ring-shaped retractor. At the position of the anterior descending coronary artery 3-4 mm below the left atrial appendage, a 6/0 damage-free silk thread was threaded, and a nylon thread with a diameter of 1.6 mm was juxtaposed and ligated together with the anterior descending coronary artery, while the other end was left outside the body for later use. The heart was placed back into the chest cavity, the air in the chest cavity was emptied, and the chest cavity was closed. 30 min after ligation, the nylon thread was carefully removed for reperfusion.

Compared with the normal lead II electrocardiogram (ECG-II), ST segment elevation in ECG-II by 0.1 mV or more 30 min after coronary artery ligation was regarded as successful ischemia. Animals that did not meet the above criteria indicated unsuccessful modeling and were excluded from the experiment.

### 3. Myocardial infarction range and determination of myocardial enzyme

The left ventricle of the heart was taken, sliced into 2 mm thick slices along the coronal plane, placed in a 2% TTC staining solution, and incubated at 37°C for 2 min in the dark for staining, COOLPIX955 digital camera imaging system was used to store digital images in a computer, image analysis system software v 4.0 was used to measure the infarction region and the whole brain area, and the myocardial infarction range (the percentage of the area of the infarction region relative to the area of the left ventricle) was calculated.

Blood was collected from the rats through the abdominal aorta and centrifuged at 3000 rpm × 10 min at 4°C, and the upper serum was taken to determine LDH by enzyme-linked immunosorbent assay.

### 4. Experimental results

### 4.1. Myocardial infarction range

Compared with the model control group, after treatment with compound B (i.v.) at 100 mg/kg, compound A (i.v.) at 100 mg/kg, and compound A (oral) at 500 mg/kg, the myocardial infarction range could be reduced by 25.2% (P < 0.05), 24.5% (P < 0.05), and 28.5% (P < 0.01), respectively; and the group of isosorbide mononitrate injection at 1 mg/kg had a decrease of 25.5% (P < 0.05). There was no significant difference between each group and the isosorbide mononitrate injection group (P > 0.05).

**Table 1 Effect of compound A/B on the myocardial infarction range in rats (x±s, n=10)**

| Group | Dosage (mg/kg) | Infarction range (%) | Improvement rate (%) |
|---|---|---|---|
| Model | - | 40.4±6.5 | |
| Compound B (i.v.) | 100 | 30.2±8.2^{∗} | 25.2 |
| Compound A (i.v.) | 100 | 30.5±11^{∗} | 24.5 |
| Compound A (oral) | 500 | 28.9±12.2^{∗∗} | 28.5 |
| Isosorbide mononitrate injection | 1 | 30.1±6.7^{∗} | 25.5 |

| | | | |
|---|---|---|---|
| Note: 1. ^{∗} P < 0.05, ^{∗∗} P < 0.01, compared with the model group. | | | |

### 4.2. Myocardial enzyme determination results

Compared with the model control group, after treatment with compound B (i.v.) at 100 mg/kg, compound A (i.v.) at 100 mg/kg, and compound A (oral) at 500 mg/kg, the myocardial enzyme LDH could be reduced by 22.2% (P < 0.05), 19.9% (P < 0.05), and 25.8% (P < 0.01), respectively; and the group of isosorbide mononitrate injection at 1 mg/kg had a decrease of 22.4% (P < 0.05). There was no significant difference between each group and the isosorbide mononitrate injection group (P > 0.05). The results were shown in Table 2.

**Table 2 Effect of compound A/B on myocardial enzyme in rats (x±s, n=10)**

| Group | Dosage (mg/kg) | LDH (U/L) | Improvement rate (%) |
|---|---|---|---|
| Model | - | 1898.8±414.7 | |
| Compound B (i.v.) | 100 | 1476.7±282.4^{∗} | 22.2 |
| Compound A (i.v.) | 100 | 1521.6±353.6^{∗} | 19.9 |
| Compound A (oral) | 500 | 1408±308.7^{∗∗} | 25.8 |
| Isosorbide mononitrate injection | 1 | 1472.7±454.5^{∗} | 22.4 |

| | | | |
|---|---|---|---|
| Note: 1. ^{∗} P < 0.05, ^{∗∗} P < 0.01, compared with the model group. | | | |

### Example 8: Protective effect of the compound of the present invention on dermal fibrosis in bleomycin (BLM)-induced scleroderm mouse model

Materials: C57BL/6 female mice, weighing 20-25 g, were purchased from Shanghai SLAC Laboratory Animal Co., Ltd. Using a random number table method, 24 mice were divided into a blank control group (1), a scleroderma model group (2), a scleroderma + compound A treatment group (3), and a scleroderma + compound B treatment group (4), with 6 mice in each group. Bleomycin (BLM) was purchased from Sigma. An H-E staining kit was purchased from Beijing Solarbio Science & Technology Co., Ltd. Compound A used in this example was in the form of a hydrochloride thereof, and compound B was in the form of a trifluoroacetate thereof.

Method: The intended injection site (area 1 cm²) of the mice was shaved using an electric shaver. The mice in groups (1) and (2) were intraperitoneally injected with PBS, the mice in group (3) were given compound A (600 mg/kg/d, dissolved in PBS) by gavage, and the mice in group (4) were intraperitoneally injected with compound B (200 mg/kg/d, dissolved in PBS). Immediately thereafter, the mice in group (1) were injected with PBS subcutaneously on the back, and the mice in groups (2) and (4) were injected with BLM (1 mg/ml, dissolved in PBS) subcutaneously on the back; and 15 min later, the mice in group (3) were injected with BLM (1 mg/ml, dissolved in PBS) subcutaneously on the back. After 28 days of continuous administration once a day, the animals were sacrificed on day 29 by cervical dislocation. Conventional iodine tincture was used to disinfect the back skin of the mice, and a local diseased skin tissue was surgically cut, fixed in 4% paraformaldehyde, embedded in paraffin, sectioned, and analyzed by H-E staining.

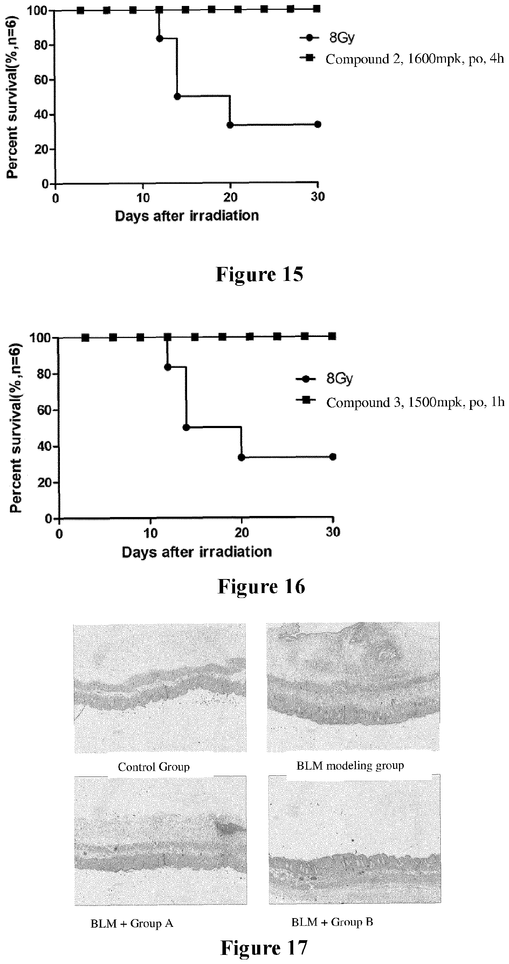

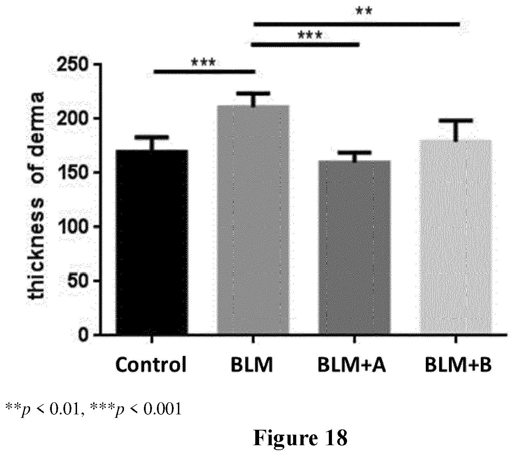

Results: As shown in Figure 17, H-E staining showed that the distribution structure of collagen fibers in the dermis in the blank control group was normal, and the fat layer was obvious. In contrast, in the BLM model group, collagen in the dermis increased significantly, and the fat layer disappeared, accompanied by obvious inflammatory cell infiltration. Compared with the BLM model group, collagen proliferation in the dermis in the compound B treatment group was improved, whereas in the compound A treatment group, the collagen thickness in the dermis was significantly reduced and the fat layer was significantly thickened. Figure 18 showed that compared with the blank control group, the thickness of the dermis in the BLM model group was reduced (p < 0.001); compared with the BLM model group, the thickness of the dermis in the compound B treatment group was reduced (p < 0.01); in addition, the treatment with compound A could better reduce the thickness of the dermis at the skin lesion site, and the difference was statistically significant compared with the BLM model group (p < 0.001).

### Example 9: Effect of the compound of the present invention on the expression level of hydroxyproline in bleomycin (BLM)-induced scleroderma mouse model

Materials: A hydroxyproline kit and an H₂O₂ kit were purchased from Nanjing Jiancheng Bioengineering Institute.

Method: Part of the skin lesion tissue of the mice was taken, and the contents of hydroxyproline and hydrogen peroxide in the skin lesion region were detected according to the requirements in the kit instructions.

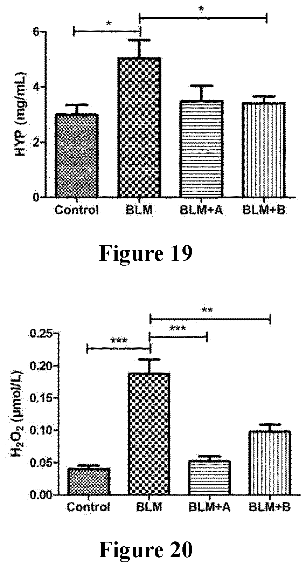

Results: In this study, the content of collagen was evaluated indirectly through the amount of hydroxyproline. As shown in Figure 19, the expression level of hydroxyproline in the skin tissue was significantly higher after BLM treatment (p < 0.05), and after treatment with compound A and compound B, the level of hydroxyproline in the skin lesion site was downregulated, wherein there was a significant difference between the BLM + compound B group and the BLM model group (p < 0.05). In addition, in this study, the level of oxidative stress in the tissue was detected by studying the concentration of hydrogen peroxide in the tissue. As shown in Figure 20, BLM could significantly increase the level of H₂O₂ in the tissue (p < 0.001), and the concentration of H₂O₂ in the skin after treatment with A and B was reduced; in addition, compared with the BLM model group, the difference was significant (p < 0.01, p < 0.001). Compound A used in this example was in the form of hydrochloride thereof, and compound B was in the form of trifluoroacetate thereof.

So far, those skilled in the art should recognize that although a number of illustrative embodiments of the present invention have been shown and described in detail herein, many other variations or modifications that conform to the principles of the present invention can still be directly determined or derived from the content disclosed in the present invention without departing from the spirit and scope of the present invention. Therefore, the scope of the present invention should be understood and deemed to cover all these other variations or modifications.

## Claims

1. Use of a compound of formula (I) or a compound selected from any one of the following Group (II) in the preparation of a medicament for use as a cerebral neuroprotective agent or a cardioprotective agent, wherein the compound of formula (I) is 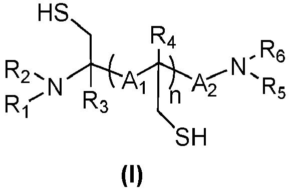 wherein
A₁ is selected from -C(O)NR⁸-, -S(O)₂-NR⁸-, -S(O)NR⁸-, and -R⁷-NR⁸-;
A₂ is selected from carbonyl, sulfonyl, sulfinyl, and substituted or unsubstituted C₁₋₆ alkyl;
R¹ and R⁵ may be the same or different and are selected from
hydrogen, and substituted or unsubstituted C₁-C₅ alkyl or
heteroalkyl;
R² and R⁶ may be the same or different and are selected from substituted or unsubstituted C₁-C₅ alkyl or heteroalkyl;
n is an integer of 0-20000;
R³ and R⁴ are independently selected from hydrogen, X, and substituted or unsubstituted C₁₋₆ alkyl;
X is selected from F, Cl, Br, and I;
R⁷ is selected from substituted or unsubstituted C₁-C₆ alkyl, and R⁸ is selected from hydrogen, and substituted or unsubstituted C₁-C₆ alkyl;
or a stereoisomer thereof or a pharmaceutically acceptable salt, prodrug and solvate thereof;
Compounds of Group (II): (R)-2-(((R)-3-mercapto-2-(methylamino)propyl)amino)-3-(methylamino)propane-1-thiol or a hydrochloride thereof (compound 1), (R)-N-((R)-3-((2-methoxyprop-2-yl)thio)-1-(methylamino)-1-oxopropane-2-yl)-2,2,3-trimet hylthiazolidine-4-carboxamide (compound 2) and (R)-2,2,3-trimethyl-N-((6R, 9 R)-3,3,12,12-tetramethyl-6-(methylcarbamoyl)-8-oxo-2,13-dioxa-4,11-dithia-7-azate tradecan-9-yl)thiazolidine-4-carboxamide (compound 3), (R)-3-mercapto-N-((R)-3 -mercapto-1-((2-(methylamino)ethyl)amino)-1-oxopropane-2-yl)-2-((2-(methylamin o)ethyl)amino)propionamide, (R)-N-((7R,10R)-6,9-dioxo-13,13,13-triphenyl-7-(trity lthiomethyl)-12-thia-2,5,8-triazatridecan-10-yl)-3-mercapto-2-(2-(methylamino)ethyl amino)propionamide, (R)-N-((R)-3,3-dimethyl-7-oxo-2-oxa-4-thia-8,11-diazadodec an-6-yl)-3-(2-methoxyprop-2-ylthio)-2-(2,2,3-trimethylimidazolidin-1-yl)propionami de and (R)-N-((R)-3,3-dimethyl-7-oxo-2-oxa-4-thia-8,11-diazadodecan-6-yl)-3-(2-methoxyprop-2-ylthio)-2-((R)-3-(2-methoxyprop-2-ylthio)-2-(2,2,3-trimethylimidazo lidin-1-yl)propionylamino)propionamide.

2. The use of claim 1, wherein the compound of formula (I) is (R)-3-mercapto-N-((R)-3-mercapto-1-(methylamino)-1-oxoprop-2-yl)-2-(methylamino)propionamide; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide.

3. The use of claim 1, wherein the compound of formula (I) is (R)-3-mercapto-N-((R)-3-mercapto-1-(methylamino)-1-oxoprop-2-yl)-2-(methylamino)propionamide or a pharmaceutically acceptable salt thereof; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide or a pharmaceutically acceptable salt thereof.

4. The use of claim 1, wherein the compound of formula (I) is (R)-3-mercapto-N-((R)-3-mercapto-1-(methylamino)-1-oxoprop-2-yl)-2-(methylamino)propionamide trifluoroacetate; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide trifluoroacetate.

5. The use of claim 1, wherein the compound of formula (I) is (R)-3-mercapto-N-((R)-3-mercapto-1-(methylamino)-1-oxoprop-2-yl)-2-(methylamino)propionamide hydrochloride; or (R)-3-mercapto-N-((R)-3-mercapto-1-(((R)-3-mercapto-1-(methylamino)-1-oxoprop-2-yl)amino)-1-oxoprop-2-yl)-2-(methylamino)propionamide hydrochloride.

6. The use of any one of claims 1-5, wherein the compound is used as a cerebral neuroprotective agent for the prevention or treatment of one or more of the following diseases or conditions: stroke, including ischemic and hemorrhagic stroke; secondary injury induced during the treatment of ischemic stroke, namely cerebral ischemia reperfusion injury; atherosclerosis, cerebral hemorrhage, hypertension, myocardial infarction, angina pectoris, coronary heart disease, myocardial fibrosis, heart disease, arrhythmia, dizziness, insomnia, etc.; varicose veins, edema and other diseases related to increased vascular permeability; senile diseases, such as Parkinson's disease, brain atrophy, and senile dementia; other brain nerve damage diseases, including but not limited to nerve injuries caused by brain trauma, sequelae of cerebrovascular sclerosis (cerebral hemorrhage, cerebral thrombosis), sequelae of encephalitis and meningitis, demyelinating diseases, ischemia, hypoxia, cerebrovascular accident, metabolic disorder, toxic effects, neurotoxic effects, surgery, iatrogenic effects, pressure, mass effect, hemorrhage, heat, chemical factors, radiation, vasospasm, neurodegenerative diseases, neurodegenerative lesions, infection, epilepsy, etc.; shock therapy, arterial bypass, thrombolytic therapy, percutaneous transluminal coronary angioplasty, cardiac surgery cardiopulmonary bypass, cardiopulmonary cerebral resuscitation, or limb replantation and organ transplantation; and systemic scleroderma or amyotrophic lateral sclerosis.

7. The use of claim 6, wherein the compound is used as a cerebral neuroprotective agent for the prevention or treatment of stroke.

8. The use of claim 6, wherein the compound is used as a cerebral neuroprotective agent for the prevention or treatment of ischemic stroke.

9. The use of claim 6, wherein the compound is used as a cerebral neuroprotective agent for acute ischemic stroke.

10. The use of claim 1, wherein the compound is used as a cardioprotective agent for the prevention or treatment of myocardial ischemia, or myocardial infarction or cardio-cerebral ischemia reperfusion injury caused by myocardial ischemia.

11. The use of any one of claims 1-10, wherein the dosage of the compound is about 9-16200 mg/kg/day.

12. A compound or a pharmaceutical salt thereof, selected from (R)-2-(((R)-3-mercapto-2-(methylamino)propyl)amino)-3-(methylamino)propane-1-thiol, (R)-N-((R)-3-((2-methoxyprop-2-yl)thio)-1-(methylamino)-1-oxopropane-2-yl)-2,2,3-trimethylthiazolidine-4-carboxamide, (R)-2,2,3-trimethyl-N-((6R, 9R)-3,3,12,12-tetramethyl-6-(methylcarbamoyl)-8-oxo-2,13-dioxa-4,11-dithia-7-azatetradecan-9-yl)thiazolidine-4-carboxamide, (R)-3-mercapto-N-((R)-3-mercapto-1-((2-(methylamino)ethyl)amino)-1-oxopropane-2-yl)-2-((2-(methylamino)ethyl)amino)propionamide, (R)-N-((7R,10R)-6,9-dioxo-13,13,13-triphenyl-7-(tritylthiomethyl)-12-thia-2,5,8-triazatridecan-10-yl)-3-mercapto-2-(2-(methylamino)ethylamino)propionamide, (R)-N-((R)-3,3-dimethyl-7-oxo-2-oxa-4-thia-8,11-diazadodecan-6-yl)-3-(2-methoxyprop-2-ylthio)-2-(2,2,3-trimethylimidazolidin-1-yl)propionamide and (R)-N-((R)-3,3-dimethyl-7-oxo-2-oxa-4-thia-8,11-diazadodecan-6-yl)-3-(2-methoxyprop-2-ylthio)-2-((R)-3-(2-methoxyprop-2-ylthio)-2-(2,2,3-trimethylimidazolidin-1-yl)propionylamino)propionamide.

13. Use of the compound of claims 1-5 or the compound of claim 12 in the preparation of a medicament for the treatment and/or prevention of radiation damage, a medicament for chemotherapeutic damage, a cosmetic, and an anti-tumor drug.
